# EUROPEAN PATENT SPECIFICATION

(11) **EP 2 131 723 B1**
(45) Date of publication and mention of the grant of the patent: **24.07.2013**
(21) Application number: 08719775.2
(22) Date of filing: 19.03.2008
(51) Int. Cl.: A61B 5/00

(54) **DEVICE FOR SUBSTANCE MEASUREMENT**
VORRICHTUNG ZUR MESSUNG VON SUBSTANZEN
DISPOSITIF POUR MESURE DE SUBSTANCE

(30) Priority: 19.03.2007 US 895518 P; 19.03.2007 US 895519 P; 19.04.2007 US 912698 P; 30.05.2007 US 940721 P; 21.06.2007 US 821230; 08.07.2007 US 948472 P; 15.10.2007 US 979904 P
(43) Date of publication of application: 16.12.2009
(73) Proprietor: Insuline Medical Ltd., 49002 Petach-tikva (IL)
(72) Inventor: PESACH, Benny, 48072 Rosh-ha ayin (IL); BITTON, Gabriel, 97279 Jerusalem (IL); WEISS, Ram, 34485 Haifa (IL); NAGAR, Ron, 62502 Tel Aviv (IL)
(74) Representative: Mintz Levin Cohn Ferris Glovsky and Popeo LLP
(86) International application number: PCT/IB2008/051050
(87) International publication number: WO 2008/114224

(56) References cited:
- EP-A- 1 611 848
- WO-A-01/01852
- WO-A-01/47408
- US-A- 6 155 992
- US-A1- 2003 040 683
- US-A1- 2004 202 576
- US-B1- 6 240 306

## Description

### BACKGROUND OF THE INVENTION

### Field Of The Invention

The present invention relates to systems to measure drug levels more accurately and in particular to glucose sensors and glucose monitoring systems wherein an additional treatment is used to improve interstitial fluid glucose measurement.

### Background

Diabetes is a very serious illness affecting millions of people. Many diabetic patients are required to measure their glucose level 5-7 times a day to maintain proper blood glucose levels. Currently, individuals intermittently measure capillary blood glucose levels by extracting a few drops of blood using what are known as finger sticks. However, intermittent use is limited in its capacity for long term glucose blood sugar level monitoring. Alternatively, continuous glucose monitoring is, allowing individuals to continuously determine and in turn maintain proper glucose levels. It has been shown that continuous blood glucose monitoring yields better short term and long term clinical outcomes.

Some of the currently available continuous glucose monitors ("CGM") for measuring the blood glucose level function in a similar manner to the capillary glucose level measured by the finger sticks. Some continuous glucose monitors include one or more sensors that are inserted or implanted into a blood vessel such as the vena cava. However, implanting a sensor into a blood vessel is a complicated process that currently requires a surgical procedure and involves potential severe adverse effects.

Conventional substance sensors are typically inserted into subcutaneous tissue. For example, the Guardian RT by Medtronic, the Navigator by Abbott and the STS by Dexcom measure glucose levels in interstitial tissue with such subcutaneous sensors. Such sensors include a selectively permeable membrane that allows glucose to flow through to an enzymatic assay that determines the relative glucose level.

Alternatively, the subcutaneous sensors include microdialysis catheter substance sensors, such as for example Menarini GlucoDay, for glucose measurement or the device of CMA Microdialysis Corp for measurement of several substances. In microdialysis, the substance molecules diffuse into the catheter membrane and flow, by use of a pump, to an external sensor, instead of inserting a sensor into the tissue, as is done in the subcutaneous biosensors. A third option currently developed by Dexcom is an implanted biosensor intended for long term use.

The more commonly used subcutaneous sensors measure the glucose levels in the interstitial fluid (ISF) to infer or estimate the corresponding blood or plasma glucose level. These sensors are inserted to the subcutaneous tissue for several days (3-7). Currently, none of the continuous glucose monitoring systems ("CGMS") are approved for independent use and they are only approved for adjunctive use because of their relatively low accuracy. CGM systems are particularly problematic during hypoglycemic episodes of low glucose levels when comparing ISF glucose measurements to glucose reading taken from blood, as they are least likely to measure the glucose levels during such episodes.

This inaccuracy is due to the fact that the glucose level at the ISF compartment differs from the blood glucose level, leading to an offset between the measured parameter and the clinically relevant parameter. The offset problem has been primarily attributed to the time required for the glucose to equilibrate between the vascular system, the ISF and the intracellular compartments. Therefore, the ISF often lags behind the blood glucose level, especially during periods of rapid changes of the glucose level, for example during a meal and/or shortly thereafter. This lag varies between 5-20 minutes and induces a significant mismatch between the blood glucose and the ISF glucose levels providing a user with misinformation that could lead to incorrect analysis and insulin dosing that would further cause an increase or decrease in the glucose levels, leading to periods of hypoglycemia or hyperglycemia.

A well documented limitation of ISF CGMS is the calibration procedure, in which sensors require a period to calibrate prior to initial use. The calibration period varies between 2 to 12 hours. During the calibration period, the ISF CGMS does not display any glucose measurements; rather, the measurements are evaluated against measured plasma glucose levels measured by using a finger stick glucose measurement.

Another limitation of the ISF CGMS is a change in the sensor's performance over time, where the initial calibration is not sufficient over time. Upon installation, the CGMS is calibrated against glucose measurement from blood samples using blood glucose meters, producing a calibration factor. However, the calibration factor may change over time due to a variety of reasons; one such reason is the variation of the glucose transport parameters between the blood and the ISF, and also variation with regard to sensor sensitivity and the sensor. A common solution to overcome this problem is recalibration of the ISF readings with regard to blood glucose readings. For example, a calibration process may be performed twice per day to improve the ISF measurement accuracy.

The end goal for CGM is to provide a closed loop monitoring system where a CGM sensor is coupled to an insulin delivery device, effectively producing a so called "artificial pancreas". However, current continuous ISF glucose monitors cannot provide sufficient glucose delivery regulation due to the lag between ISF glucose levels and blood glucose levels and the overall inaccuracy of the blood glucose estimation.

An apparatus according to the preamble of claim 1 is known from US 2003/040683.

### SUMMARY OF THE INVENTION

As such, there is a need for a system and a method for accurately inferring blood glucose levels from the readings of interstitial fluid ("ISF") glucose levels.

The present invention overcomes the above deficiencies of the background by providing a system according to claim 1. The introduction of a treatment element improves the performance of the ISF glucose sensor, through one or more of a plurality of pathways that allow stable and accurate prediction of blood glucose levels based upon measured glucose levels from interstitial fluid.

Estimating blood glucose levels from ISF glucose levels is improved in some embodiments of the present invention by one or more of the following effects: reducing the plasma to ISF delay, reducing the variability of the delay, reducing the time to steady state in tissue where the sensor is placed, stabilizing the calibration process, stabilizing the calibration period, reducing calibration period, minimizing the error of current ISF sensors and/or stabilizing the parameters relative to the ISF glucose levels. Accurate estimations of blood glucose levels are needed to regulate the blood glucose level of diabetic patients. Correct blood glucose estimation may also enable automatic control of blood glucose levels by combining the device for glucose level estimation with an insulin delivery device and preventing or at least reducing to a minimum hyperglycemic and hypoglycemic events, such that the insulin forms a treatment material for treating a disease associated with the substance being measured, in this case glucose.

Application of a treatment to the tissue in which the sensor is implanted and/or surrounding the sensor reduces the variability of delay between different sensors, reduces time to steady state measurements in tissue area (thereby reducing the sensor stabilization period), reduces the calibration period required prior to measurement implementation, allows a sensor to accurately measure ISF glucose levels for the individual thereby providing greater personalization, and reduces intra-patient variability.

Although the present application refers to diabetes and glucose monitoring, it can be understood by one skilled in the art that such references are provided for exemplary, illustrative purposes only and are not intended limit the scope of the present invention. The system and method of the present invention may be similarly applied to other substances which may be measured with high accuracy in a non-blood fluid, in relation to measurements in the blood. Another non-limiting example of an illustrative application of the present invention includes measuring cholesterol levels or the like for monitoring anomalies or conditions such as atherosclerosis. Also it should be noted that although the term "patient" is used herein, the present invention may be used with any subject or user.

The present invention features one or more substance measuring devices, for example biosensors, sensors or the like, to analyze and provide details regarding a substance of interest that is being measured. For example, a glucose monitor is used to measure the level of glucose in tissue or fluid. The substance measuring device may analyze the substrate tissue and/or fluid to provide information regarding different parameters relating to the substance being measured. The substrate fluid or tissue may be analyzed in vitro or in situ by obtaining a sample for analysis in various ways, for example extraction, catheter, microdialysis catheter, implantation, implanted sensor having a membrane exposed to the ISF, iontophoresis or the like.

In some embodiments, the substance measuring device then draws a sample of ISF through the catheter and measures different parameters relating to the substance content in the drawn ISF. In some embodiments, as with regard to the non-limiting examples of Guardian RT by Medtronic, the Navigator by Abbott and the STS by Dexcom, at least a portion of the measuring device is inserted into subcutaneous tissue and includes a selectively permeable membrane that allows glucose to flow through to an enzymatic assay that determines the relative glucose level.

In some embodiments, the present invention provides for a device that continuously measures ISF glucose levels and enables a more accurate estimation of blood glucose levels, through the application of one or more treatments to the tissue into which the sensor is inserted and/or surrounding the sensor.

In some embodiments, the present invention provides for a device for measuring relative levels of at least one or more substance and/or chemical in a tissue, for example one or more of glucose, cholesterol, hemoglobin, or the like for improving the effectiveness of such substance measurement.

The substance sensor is inserted into a tissue region of interest in which the substance level is to be measured. The substance sensor obtains a sample of the substance by diffusion from a volume around the opening or the active area of the substance sensor, called "measured tissue region". The substance sensor can be inserted into the subcutaneous tissue, as usually done for measuring the ISF glucose level. In such embodiments, the treatment can be applied to the measured tissue region, to expose the vicinity of the measured tissue region to an energy source such as radiation, heat, mechanical vibrations, suction, massaging, acoustic stimulation (e.g., ultrasound), electrical stimulation, infusion of an additional substance(s), or any combination of the above to improve and/or stabilize the pharmacokinetic of the substance and/or reduce its transport time between the vascular and ISF comportments at the tissue region.

For example, in the case of glucose monitoring, plasma glucose level arc estimated based on the measured ISF glucose readings. Many of the models that were developed for showing the relation between the plasma and ISF glucose and/or for estimation of the plasma glucose from ISF glucose assume two or three compartments with constant transport rates coefficients of the glucose or other substances between the compartments, such as disclosed by Rebrin and Steil, Diabetes Technology and Therapeutics, 2, 3 461-472 (2000), or by Facchinetti et al., Journal of Diabetes Science and Technology, 1, 5 617-623 (2007) and many others. Facchinetti et al., suggest that the Linear Time Invariant (LTI) model is not accurate enough for serum glucose prediction based on ISF glucose levels. They show that the estimation of plasma glucose level using a continuous ISF glucose sensor readings based on the LTI model may be improved by repeated recalibration of the ISF readings. The recalibration process compensates for inaccuracies of the LTI model induced by additional variations of the substance transport process, which are not covered by the LTI or similar models. The LTI model used by the above two papers may be described by the following equation: 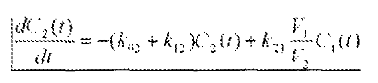 where *C*₁(*t*) represents the plasma glucose concentration and *C*₂(*t*) represents the ISF glucose concentrations. Similarly, *V*₁ represents the plasma volume while V₂ is the ISF volumes, and *k*ᵢⱼ denotes the transfer rate from compartment j to compartment i. Facchinetti rewrites it as the following integral equation: 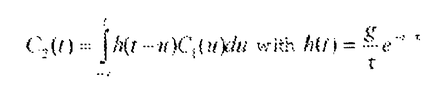 where g and τ are the "steady state gain" factor and the transport time constant respectively, given by g = (*k*₂₁*V*₁/*V*₂)τ and τ= 1/(*k*₀₂ + *k*₁₂).

However, the different transport rates, *k*ᵢⱼ, are dependent on different parameters that are not accounted for in the equation, for example tissue perfusion and temperature. Changes in such parameters effectively change the transport coefficient, *k*ᵢⱼ, in turn changing either g and/or τ consequently leading to inaccuracies in the plasma glucose estimation *C*₁(*t*) based on the measured *C*₂(*t*).

Some embodiments of the present invention provide for a device that improve the transport process of the measured substance between the blood and ISF compartments as depicted in the LTI estimation. In some embodiments, improvement is provided through better control of one or more parameters of the process. By controlling the transport process, the estimated plasma glucose levels are more accurately determined from the ISF glucose levels.

In some embodiments, the present invention relates to a device which provide an improved transport process of the sample from the ISF source into the substance sensor's membrane (or other measuring and/or detecting component).

In some embodiments, the present invention relates to improved control of the transport process by introducing a treatment protocol in the vicinity of the sample source site, allowing the stabilization of the transport coefficients k and therefore of the coefficients g and τ.

In some embodiments, the present invention relates to a treatment element that applies a treatment to a tissue region in the vicinity of a substance sensor, to reduce the variability of transport coefficients k values and/or g and τ.

In some embodiments, the present invention relates to a treatment element that applies a treatment to a tissue region in the vicinity of a substance sensor, to shorten the transport time constant τ. For example, shortening the transport time constant improves the estimation accuracy of the blood glucose level and is conducive for closed loop applications with substance measurement having artificial control, as in the case of glucose regulation.

In some embodiments, the present invention relates to a treatment element that applies a treatment to a tissue region in the vicinity of a substance sensor, to reduce the variability of g and τ and to shorten the transport time constant τ. For example, the treatment may reduce the variability of the tissue temperature and/or the variability of the tissue blood perfusion in order to reduce the variability of g and τ, and more during the events of substance measurement. The treatment may also increase the tissue blood perfusion to a known level of blood perfusion in order to reduce the variability of g and τ and to shorten the transport time constant τ, again and more during the events of substance measurement.

The treatment may also expand the tissue capillary pores to a known level in order to reduce the variability of g and τ and to shorten the transport time constant τ, again and more during the events of substance measurement.

In some embodiments, the present invention relates to for a plurality of treatments. The treatment can be applied for one or more of the following: control the k, g and τ parameters, reduce delay, reduce variability of delay, reduce time to steady state in tissue area, reduce time for calibration period, reduce variability between patients, or reduce variability within a single patient. Therefore, leading to predictable prediction of blood glucose levels based on ISF glucose levels. The treatment type includes, but is not limited to, heating, modifying temperature, nociceptive axon reflex protocol, massaging, mechanical vibration, acoustic vibration, ultrasound, suction, infusion or applying to the skin of an additional substance or chemical, applying a low electric field, applying a low magnetic field, light irradiation, infrared ("RF") irradiation, microwave ("MW") irradiation, or the like treatment modality.

In some embodiments, any one or a combination of the treatment protocols may induce expansion of the capillary pores, improving the capillary permeability and the transport rate of the measured substance between the blood and the ISF.

In some embodiments, any one or a combination of the treatment elements and their respective protocols may lead to reduced delay between ISF glucose measurements and blood glucose measurements. Therefore, the ISF glucose sensor according to the present invention more accurately reflects the blood glucose levels faster than state of the art ISF sensors. Reduced delay may be realized in reduction or improvement in the elapsed time required for an ISF sensor to equilibrate and register the same or at least similar glucose measurements as recorded in the blood glucose levels.

In some embodiments, the respective treatment protocols cause the desired effects through one or more of various chemical or biological pathways for example increased vasodilatation, improved diffusion across blood barrier, improved capillary permeability, improved capillary diffusion, improved cellular metabolism, improved tissue metabolism, improved local solubility, changed local membrane microstructure, improved local permeability, improved facilitative diffusion, improved carrier protein metabolism, improved diffusion mediated by a carrier protein, changed local pressure gradient, changing the local ionic gradient, up-regulation or down regulation of local gene expression, up-regulation or down regulation of local transcription, up-regulation or down regulation of local translation, changed local enzymatic activity, changed local lymphatic activity, and/or improving the Foreign Body Response of the tissue to the sensor inserted into the tissue, by coating of the sensor with one or more different molecules or the like.

In some embodiments, any one or a combination of the treatment elements and their respective protocols may lead to a reduction in the variability of the delay between ISF glucose measurements and blood glucose measurements. Therefore, the ISF glucose sensor according to the present invention is more consistent in the ISF to glucose delay and therefore the delay is but a constant factor that allows for continued provision of more accurate predictions of the blood glucose levels based on ISF glucose readings. Such control allows for prediction of glucose trends for the individual patient, thereby improving blood glucose and insulin dosage management. Reduced variability in ISF to plasma delay may be realized in that the delay is consistent and predictable, as is the time required for an ISF sensor to equilibrate and register the same glucose measurements as recorded in the blood glucose levels. For example, variations in the delay of the ISF glucose level readings and the determination of blood glucose level, and/or the determination of other analyte levels, impose a major difficulty for algorithms that control glucose levels through the administration of insulin, and may induce a large error in the resulting glucose level regulation.

In some embodiments, any one or a combination of the treatment elements and their respective protocols may lead the ISF sensor according to the present invention to reduce the time to reach equilibrium or steady state within the measured tissue area, and/or the time required to calibrate or the number of calibrations required within the measured tissue area. Therefore the ISF glucose sensor according to the present invention may be functional in a short period of time, wherein useful ISF readings may be available faster than in current state of the art CGM systems.

In some embodiments, any one or a combination of the treatment elements and their respective protocols may lead the ISF sensor, according to the present invention, to reduce the variability between patients such that it is adjustable to the patient's needs. Some of the discussed treatments reduce the glucose transport delay and stabilizes the transport coefficients, such that the variability between patients and within the same patient is reduced. The treatment results can be the same, however, the method by which the results are obtained are personalized to an individual user. The respective treatment protocols cause the desired effects by various chemical or biological pathways as described herein.

In some embodiments, any one or a combination of the treatment elements and their respective protocols may lead the ISF sensor, according to the present invention, to reduce the variability of one or more measurements within a user such that it is specific and predictable for the individual user. Some of the discussed treatments reduce the glucose transport delay and stabilize the transport coefficients, such that the variability between patients and within the same patient is reduced. For example, a user's individual limits are known and predictable (e.g., within a range), thereby enabling the treatment protocol to be tailored to the user. The respective treatment protocols cause the desired effects by various chemical or biological pathways as described herein.

In some embodiments, treatment may be applied to the source tissue from where a sample is being analyzed with the substance sensor according to the present invention. The treatment can be applied at the source site, or in its vicinity, to cause the desired effect.

In some embodiments, the present disclosure relates to various methods of obtaining a sample for analysis for example extraction, use of a catheter, use of a microdialysis catheter, implantation, use of an implanted sensor having a membrane exposed to the ISF, iontophoresis or the like. The substance sensor is preferably placed subcutaneously within a catheter placed at the sample source tissue region.

The measured tissue region can be one of the skin layers or the subcutaneous tissue or deeper tissue elements within any organ or viscera, for example when implanted.

The device according to some embodiments of the present invention may be securely adhered in place over the skin using fastening agents such as adhesive, glue, strap or the like. Securing the substance measuring device according to the present invention prevents its movement, and also reduces the likelihood of discomfort and/or accidental removal.

In some embodiments, the device according to the present invention may be coupled to a plurality of auxiliary units that perform various functions. An auxiliary unit may communicate to the substance measurement device according to the present invention by various communication protocols wired, wireless, cellular, IR, RF, or the like communication protocols. Auxiliary units used may be coupled or decoupled to the device according to the present invention, allowing a user to continuously use the device of the present invention with or without the auxiliary unit. The auxiliary unit to the system and device according to the present invention may include but is not limited to a processing unit, additional substance sensing device, data storage unit, treatment device, drug delivery device, display unit, audio unit, communication unit, power supply, PDA, computer, cellular phone, disposable device or the like.

In some embodiments, the substance sensor or the auxiliary unit's electronic processing unit operates according to a predetermined protocol or algorithm and/or any additional inputs of sensors to optimize the applied treatment effect. In some embodiments, the auxiliary unit electronic processing unit communicates with the treatment device processing unit, which operates according to a predetermined protocol or algorithm. In some embodiments, the device is neither controlled by the auxiliary electronic processing unit nor has any communication with it, yet applies the treatment continuously.

In some embodiments, an auxiliary unit can be attached externally to improve the user's comfort. The auxiliary unit can be disposed in a bag, a pouch, a case, or a belt adaptor containing an auxiliary unit as devices used for carrying insulin pumps. In such a case, the sensor may be wired or wirelessly connected to the auxiliary unit which gets the sensor readings, while the wires of the treatment device are connected to the auxiliary unit, which may be disposed in the canying case. The auxiliary unit or the carrying case can also include a switch for manual start of the treatment or indicators for indicating that the treatment is applied or indicators that the battery power is adequate, too low or indicators that a problem occurred with the treatment, such as wire disconnection, etc. The switch or indicators, or a portion thereof, can be disposed also on the reusable unit or disposable unit or on auxiliary units.

In some embodiments, the devices by the present invention can have short range RF or IR communication with a data management and control unit, such as a Personal Digital Assistant ("PDA") computer, to a personal cellular telephone or other mobile communication device, or to an application specific data managing device that supports managing drug therapy. In case of glucose monitoring, a data managing device can obtain the glucose readings from the substance sensor through data communication or use also reading of glucose sensing strips calibration. The data managing device may also obtain information about previously consumed carbohydrates and other food or drinks. The data managing device can also retain patient history and relevant parameters, such as weight, BMI, insulin resistance etc.

The data managing device can also calculate the optimal required amount of insulin. In some embodiments, the data managing device can set the optimal tissue treatment or stimulation profile, as the algorithms described in the present application, directly or through the auxiliary unit. The additional information stored in the PDA or other data managing device, such as food intake can be used for the algorithm of the applied treatment. For instance, after meal intake a more rapid glucose rise is predicted; the device can use that information to decide to apply a treatment or stimulation to the measured tissue vicinity to shorten and or regulate the delay associated with glucose transport from the blood to the ISF, which may induce larger errors of glucose reading at faster glucose variations. The treatment device may also transmit tissue parameters measured by sensors disposed thereon to the data management unit (which may also be or include the control unit to form a "data management and control unit") as additional information for the therapy calculation or history for future statistics and data analysis. In some embodiments, the data management and control unit may include one or more of a switch for manual start of the treatment, indicators for indicating that the treatment is applied, indicators that the battery power is adequate, too low or indicators for determining if a problem occurred with the treatment, such as wire disconnection, etc.

In some embodiments, a controller that controls the treatment element can be used. The controller functions to perform one or more of the treatment protocols, keep a history of the treatment, control the function of the treatment element, facilitate integrated function of the treatment element and corresponding sensor or other device, and/or to perform any controlling function known and accepted in the art.

For example, the controller and sensor may function together to cause tissue treatment according to a temperature change protocol. The temperature controller can set the temperature of the measured tissue region to a constant temperature based on reading from a sensor. The temperature controller may be used to set a profile of the temperature dynamics at a known rate, temperature stabilization at a known period and ending the profile with a return to the natural tissue temperature. Such a protocol may be induced by application of a heating treatment element that heats the vicinity of the tissue region into which the substance sensor is inserted. Execution of such a protocol may be repeated periodically every time a substance measurement is required.

In some embodiments, the treatment protocol may be applied to a larger region than the tissue region of substance measurement. Doing so may improve blood perfusion also in the vicinity of the measured tissue region and by way of a further increase of substance transport absorption by increasing the available high blood perfusion volume. The treatment profile can be applied to a region smaller than the measured tissue region, for example to save battery life. The treatment protocol may include cooling of the measured tissue region to a certain treatment. For instance, in some cases where it is desired to calibrate the sensor operation within a treatment range, cooling can be used to achieve such a calibration treatment profile.

In some embodiments, the present invention provides for a treatment element that applies heat to control glucose transport parameters and therefore produce reliable ISF reading results. For example, heat is and applied to control g and τ, which are the "steady state gain" factor and the transport time constant respectively as described in greater detail above. Heat can be applied to the sample tissue source using various heating element for example resistors, printed resistors, PCB (printed circuit board) boards with heating element, or the like.

In some embodiments, the treatment element may be manufactured by printing technologies, for example to form a PCB based treatment element. A heating treatment element may have a thickness of from about 0.1 to about 0.5mm or in other cases from about 0.5 to about 2mm. PCB treatment elements may be flexible and therefore more comfortable for the user.

In some embodiments, the heating element may include a controller that controls the heating element; for example providing coarse control such as on or off options, or more fine control for example including the ability to increase temperature or power from the element. This heating element control is used to stabilize the skin temperature to the required temperature according to the treatment algorithm or protocol being used. The temperature may range from about 32 °C to about 40°C in order not to irritate the skin on the one hand and to have a sufficient effect on the tissue on the other hand. Higher temperatures can be used for short periods. In some embodiments, other temperatures can be used. Conventional temperature stabilization algorithms art may be used using and can be executed by controllers/processing units or ASICs to prevent skin or tissue damage.

An optional treatment protocol uses controlled heating induce neural thermal stimulation such as the nociceptive axon reflex, causing vasodilatation at a distance of up to about 30mm form the heat source, as disclosed in W. Magerl et. al. Journal of Physiology 497.3 837-848 (1996). The thermal stimulation treatment protocol, such as heating for one minute, may evoke sustained vasodilatation for a period of few minutes. Thermal stimulation protocol may be customized or calibrated to a user to obtain the wanted vasodilatation results according to the users' individual neural or nociceptive axon reflex parameters. Calibration process may be used to ensure that the thermal stimulation protocol is function properly avoiding a user's secondary effect, such as a result of the axon reflex, as reported in Belinda et. al. J. Physiol. 572 3 pp 821-820 (1996).

As a non-limiting example, the axon reflex protocol may call for heating to a temperature of 37-43°C that is applied for short periods of 2-60 seconds for a predetermined period or according to substance sensor readings to evoke vasodilatation that improves the substance pharmacokinetics and/or transport process in the measured tissue region. For example, an optional axon reflex protocol may call for heating the source tissue area to temperature of 39.5°C that is applied for short periods of 2-60 seconds and is applied according to the needed measurement frequency, such as a measurement every 5-60 minutes, thereby evoking vasodilatation that improves the substance pharmacokinetics in the measured tissue region.

In some embodiments, the present disclosure relates to a calibration process in which ISF glucose level is calibrated against serum or blood glucose levels more than once. The calibration protocol between ISF levels and blood levels is calibrated at a period of time for example according to one or more of initial use, set frequency, varying frequency, daily, hourly, intermittently, irregular frequency, chaotic frequency, randomized frequency, user defined schedule or the like time frame. The calibration protocol is undertaken to ensure that the ISF measurements accurately reflect serum glucose levels.

State of the art CGMS allow for a calibration period after the sensor is placed until it starts to provide glucose readings, which ranges between 2-12 hours Belinda et. al. J. Physiol. 572 3 pp 821-820 (1996). The CGMS according to the present invention reduces the calibration period. The calibration period is needed to allow the tissue in the vicinity of the insertion point to stabilize. An optional CGMS according to the present invention increases local blood perfusion, reduces local inflammation, and/or enhances other processes that may shorten the tissue stabilization period, thereby reducing the sensor's calibration period.

In some embodiments, the tissue in the vicinity of the sensor's insertion point is treated with a treatment element heat. The applied treatment is used to reduce inflammation, biofouling of the sensor and to enhance healing of the wounded tissue region, such that the sensor's tissue vicinity will stabilize faster. In some embodiments, part of the calibration process of the sensor includes changing the temperature of the tissue in the vicinity of the sensor's insertion point during periods of relatively constant glucose levels. This process provides data specific to the tissue location and the sensor. The sensor's sensitivity as well as the tissue transport properties at different temperatures may be determined. , this data may be analyzed and stored to be used later to improve the accuracy of the blood glucose estimation using the sensor readings.

The device according to the present invention may include disposable and reusable parts. In some embodiments, any of the main device portions may be either disposable or reusable, or both, or in any combination thereof. Any auxiliary parts may be disposable or reusable, or both, or in any combination thereof. Any device portions that are in contact with tissue or fluid are disposable, for example needles, adhesives, catheters, batteries, or the like.

In some embodiments, one or more properties of the individual treatment or stimulation sources, for example: one or more of amplitude, phase, frequency, or the like, parameters based on the chosen treatment modality, the combination of treatment or stimulation sources, the relative ratio and timing between the various treatment or stimulation sources, may be controlled by a processor in order to achieve a desired response of the measured tissue region. The treatment elements may be adjusted according to the chemical/physical properties of the measured tissue region and of the substance to be measured.

In some embodiments, tissue treatment may be applied according to a protocol that is determined according to the treatment element or elements used. For example treatment protocols may be continuous and/or intermittent, as necessary to evoke the desired effect.

The decision to start or stop a treatment or stimulation protocol may be derived by analyzing the sensor data with a processor and then deriving, according to an algorithm, a decision to start or stop the treatment or stimulation according to the required protocol.

Tissue treatment or stimulation protocols may be initiated according to a predetermined schedule. Non-continuous or intermittent operation may be used for power saving, to prevent undesired physiological responses to the treatment or stimulation such as adaptation of the neural response to stimulations.

In some embodiments, the decision to start a treatment is done by analyzing the glucose readings with a processor that identifies rapid glucose variations, for example during food intake. During such rapid fluctuations, the delay of the ISF glucose level comparing to the blood glucose level may induce larger errors in the ISF glucose reading. To overcome this problem, a treatment, using a treatment element according to the present invention, may be applied for a given time period or until the glucose variations decrease. The processor may identify variations, fluctuation or trends in the glucose levels and apply an appropriate treatment to improve the accuracy of postprandial glucose readings.

In some embodiments, the processor may identify specific meals, for example, breakfast, lunch or supper, according to glucose variations and/or additional information, for example time of the day, and apply the appropriate treatment to improve the postprandial glucose readings accuracy. Such specific meals can be identified not only according to the intake of any calories, but can also be identified according to a usual caloric intake. For example, if the largest meal of the day typically occurs in the evening within a certain time period, information regarding this typical meal pattern is incorporated by the processor in order to select and/or apply the appropriate treatment.

The decision to start a treatment is made by analyzing data obtained from at least one and more a plurality of temperature sensors in relation to one another and with respect to a given temperature range. This analysis is used to ensure the proper and accurate functioning of the ISF glucose sensor and that the relative temperatures obtained from a plurality of sensors allows the glucose sensor to function properly. Accordingly, tissue treatment may be used to ensure that the sensor is functioning within an appropriate temperature range. For example, one or more of the temperature at the ISF glucose sensor and/or the temperature of the tissue in the vicinity of the sensor and/or the skin temperature and/or ambient temperature is measured by at least one temperature sensor. The associated processor then identifies that the sensed temperature is deviating from a set temperature or temperature range, which can induce variations to the readings of the sensor, for instance by variation of the delay of the ISF glucose level comparing to the blood glucose level, and consequently induce errors in the blood glucose estimation. The treatment may applied for a given time period or until the temperature variations stabilize.

The decision to start a treatment may be triggered during any period, scenario or time frame where accurate blood glucose estimation from ISF glucose readings is susceptible to error, one or more of inter-daily fluctuations, food ingestion, physical activity, physical inactivity, and peripheral shut down during hypoglycemia resulting in low peripheral blood perfusion. These time frames may be identified by one or more of a variety of sensors, for example motion sensors, such as an accelerometer or a local blood perfusion sensor in the vicinity of the insertion point. Such time frames may be realized by analyzing the glucose readings of the sensor with a processor. The treatment may be applied for a set time period according to a predefined protocol, or until the processor identifies that those parameter(s) or combination of parameters result in readings that are no longer susceptible to error.

In some embodiments, the processor may decide to start a treatment during specific situations or time frames, in which a more accurate glucose reading is required, such as for example measuring the glucose level in the middle of the night to identify hypoglycemia or before the morning to identify a typical increase in glucose by dawn phenomenon. Another example is analyzing the glucose readings by the processor to identify low glucose levels or a rapid glucose decrease that may lead to hypoglycemia. The treatment may be applied for a set time period, until an accurate glucose reading is taken, or until the glucose level goes back to normal.

In some embodiments, the treatment may include application of an additional substance such as a fluid, chemical, compound or the like that may induce vasodilatation. A chemical may be infused into the vicinity of the measured tissue region, such that the additional substance modifies the measured substance pharmacokinetic and/or local blood perfusion with or without the creation of a chemical or other reaction between the two substances. This effect is not necessarily due to a chemical reaction between the measured substance and the additional substance. In some embodiments, the additional substance improves local blood perfusion in the vicinity of the measured tissue region and accordingly, reduces the delay of the measured substance transport from the blood system to the substance sensor. This effect may be additive or synergistic to the above described forms of stimulation. For instance, glyceryl trinitrate, which induces vasodilatation, can improve blood perfusion in the measured tissue region and improve the substance transport from the blood system into the substance sensor. Another example is capsaicin that stimulates a neural response through the VR1 receptor and produces a similar response as to thermal stimulation.

In some embodiments, of the present invention, a device for supplying energy to a tissue region (or infused region vicinity) may be configured to monitor and control the properties of the treatment or stimulation sources (such as one or more of amplitude, phase, intensity, frequency, etc.). The monitoring information can be provided to a controller ("controller" or "processing unit") that uses the information to reduce the variability of transport process of the measured substance molecules between the compartments, such as the blood, ISF and intracellular compartments, in the measured tissue region between different measurement events of the substance. One possible method to reduce the time constant of the substance transport between the blood and the ISF is to increase local blood perfusion in the measured tissue region vicinity. Moreover the variability in the delay time may be reduced as well. The parameters governing the substance transport between the blood and ISF compartments such as the substance transport coefficients depend on external parameters; fixing those parameters to a constant value reduces the variability of the g and τ, as discussed above. One possible method for such a reduction is to heat the measured tissue region vicinity. Heating to the same temperature will increase local blood perfusion of the measured tissue region vicinity and also reduce the blood perfusion variability, since without heating the local blood perfusion of the measured tissue region vicinity strongly depends on the ambient temperature.

In some cases, the transport of the measured substance from the ISF compartment at the measured tissue region into the substance sensor, which usually goes through a membrane, also depends on the temperature of the substance sensor and the measured tissue region vicinity. Heating may also slightly opens the capillary pores to a certain extent, so the substance molecules will be able to diffuse between the blood and the ISF more rapidly. Thus, heating at a predetermined temperature reduces the variability of the transport of the measured substance molecules from blood to the ISF and also may reduce the variability of the transport of the measured substance molecules from the ISF into the substance sensor. Without heating, this process depends on ambient temperature.

In some cases, the measurement process of the substance by the substance sensor itself depends on the temperature. For example the enzymatic reaction involved in regular glucose oxidase based glucose measuring systems depends on temperature. By monitoring the treatment or stimulation parameters it is possible to control the treatment in a way that does not induce a response of the sensor itself to the tissue treatment. In some embodiments, where the measured tissue region temperature is regulated, the temperature induced sensor variations are reduced.

In some cases of substance sensors, such as glucose sensors based on the glucose oxidase enzyme, the enzyme reaction requires also a minimum level of oxygen. Thus, increasing the local blood perfusion can also improve and stabilize the oxygen supply to the enzyme. In other cases, where other substances originating from the blood or lymph or delivered exogenously are required for sensor activity, modifying local tissue perfusion will similarly hasten the equilibration process and facilitate more stable and reproducible substance delivery to the sensor.

In some embodiments, the device can be configured to monitor properties of the measured tissue region vicinity (such as temperature). Based on such monitoring, the information can be provided to the controller that uses the information to improve the treatment to the measured tissue region vicinity and to reduce variability of the substance transport and measurement processes.

In some embodiments, the temperature of the region adjacent to the measured tissue region is regulated at each measurement point in time so that the temperature at the measurement time is the same as the temperature at the most recent calibration time. Using this technique the accuracy of the sensor can be improved. The temperature regulation can begin for a short period, such as required for temperature regulation of the measured tissue region or for stabilization of the substance transport process, before a substance measurement is performed. The temperature of the region adjacent to the measured tissue region can be regulated for longer periods, but there may be an additional cost related to the energy source volume and weight. Therefore, in some embodiments, for minimization of the energy source size the heating period is optimized in relation to the frequency of substance measurement and the period of physiological stabilization of the measured tissue region vicinity.

In some embodiments, the tissue treatment or stimulation device may be triggered manually by the user. The user may activate the treatment device or devices before or during or after the period in which one wishes to get a faster and more accurate reading. In such embodiments, triggering may be performed by pressing a button or a sequence of buttons on the tissue treatment device. In some embodiments, in case of communication between an auxiliary unit and the treatment device, the treatment can be triggered manually by pressing a button or a sequence of buttons on that auxiliary unit. For example, in case of an insulin pump communicating with a continuous glucose sensor, the pump may have a special button for triggering an "accurate measurement" reading. In some embodiments, the pump processor can decide automatically using a predetermined algorithm or conditions, such as discussed above, to initiate the tissue stimulation. In some other embodiments, the processor of the auxiliary unit can decide automatically using a predetermined algorithm or conditions, such as discussed above, to initiate the tissue stimulation.

In some embodiments, the substance sensor unit includes at least one of the following treatment or stimulation sources or at least one combination of two or more such sources from the following: a heat source (e.g., a heat resistor), a suction port activated by a pump (for example), a mechanical vibration source, an ultrasound source, an ultrasound transducer, a light source, an optical fiber, a massaging element, catheter for infusion of additional substance and/or a combination of at least two of sources of heat, vibrations, suction, ultrasound, light, infusion of additional substance and massaging.

The substance measurement data obtained with the system and method according to the present invention may be used to control and determine further activities related directly or indirectly to the measured substance. Drug delivery may be automatically or manually altered based on the substance measurement. For example, if the ISF glucose sensor according to the present invention registers a high glucose reading, the reading is communicated to an auxiliary unit, for example a drug delivery unit, that determines the insulin dosage required to stabilize and control the current glucose levels. Measured glucose levels may be communicated to an auxiliary unit that has both the capabilities of drug delivery and tissue treatment. For example, a high glucose reading communicated to at least one or more auxiliary units is used to determine both appropriate tissue treatment and insulin dosage to optimize the glucose level control.

Any of the methods or algorithms described herein may be implemented, partially or completely, as software, firmware, hardware or a combination thereof.

Unless otherwise defined, all technical and scientific terms used herein have the same meaning as commonly understood by one of ordinary skill in the art to which this invention belongs. The materials, methods, and examples provided herein are illustrative only and not intended to be limiting.

Implementation of the method and system of the present disclosure involves performing or completing certain selected tasks or steps manually, automatically, or a combination thereof. Moreover, according to actual instrumentation and equipment in some embodiments of the method and system of the present disclosure, several selected steps or H d stages could be implemented by hardware or by software on any operating system of any firmware or a combination thereof. For example, as hardware, selected steps of the invention could be implemented as a chip or a circuit. As software, selected steps of the disclosure could be implemented as a plurality of software instructions being executed by a computer using any suitable operating system. In any case, selected steps of the method and system of the disclosure could be described as being performed by a data processor, such as a computing platform for executing a plurality of instructions.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the embodiments of the present invention only, and are presented in order to provide what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

Figure 1 illustrates an exemplary device for measuring the level of a substance in the tissue combined with a heating device, according to some embodiments of the present invention.

Figures 2A-I show graphs illustrating exemplary results of measuring the level of glucose in the tissue with and without heating of the vicinity of the measured tissue region.

Figures 3A-C illustrate exemplary substance sensors for measuring the level of a substance in the tissue combined with a heating element.

Figure 4 illustrates an exemplary device for treatment of a tissue region combined with a substance sensor for measuring the level of a substance in the tissue made of disposable part and reusable part, according to some embodiments of the present invention.

Figure 5 illustrates an exemplary device for measuring the level of a substance in the tissue combined with a mechanical vibrating element attached to the skin around the catheter, according to some embodiments of the present invention.

Figure 6 illustrates an exemplary device for measuring the level of a substance in the tissue combined with a mechanical vibrating element attached to the skin around the catheter, according to some embodiments of the present invention.

Figure 7 illustrates an exemplary device for measuring the level of a substance in the tissue combined with a massaging element that massages the skin around the catheter, using air cushion, according to some embodiments of the present invention.

Figure 8 illustrates an exemplary device for measuring the level of a substance in the tissue combined with a suction element that affects the skin around the catheter, according to some embodiments of the present invention.

Figure 9A-B illustrates an exemplary device for measuring the level of a substance in the tissue combined with an optical radiation source irradiating the skin close to the catheter, according to some embodiments of the present invention.

Figure 10 illustrates an exemplary device for measuring the level of a substance in the tissue with an acoustic treatment or stimulation of the skin close to the catheter, according to some embodiments of the present invention.

Figure 11 illustrates an exemplary device for measuring the level of a substance in the tissue combined with a catheter for substance delivery, according to some embodiments of the present invention.

Figure 12 illustrates an exemplary device for measuring the level of a substance in the tissue combined with a general treatment device, according to some embodiments of the present invention.

Figures 13A-C illustrate exemplary devices having disposable and reusable portions.

Figure 14A illustrates an exemplary device for measuring the level of a substance in the tissue combined with a U shaped heater, according to some embodiments of the present invention

Figure 14B illustrates an exemplary device for measuring the level of a substance in the tissue combined with a circular thin heater, according to some embodiments of the present invention.

Figures 15A-B are flow charts of exemplary methods for initiating tissue treatment based on sensed data.

Figure 16 is a flow chart of an exemplary method for initiating tissue treatment based on sensed data .

### DESCRIPTION OF THE INVENTION

Embodiments of the present invention relate to devices for improving, modifying and/or stabilizing kinetics and /or modifying transport of a substance measured by a sensor placed in the tissue and more specifically by ISF sensors, for measurement of glucose levels although for measurement of one or more other or additional substances. Some of the embodiments apply additional treatment or stimulation to the vicinity of the substance measurement site.

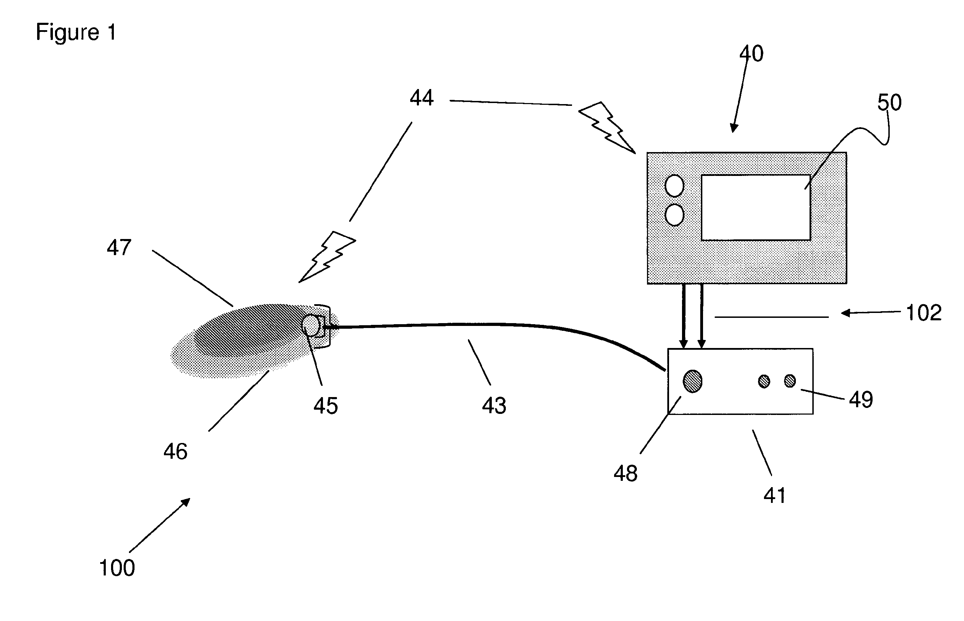

Figure 1 depicts an exemplary substance sensor device including a substance sensor apparatus 100, for example in the form of a continuous glucose monitor apparatus, along with an auxiliary apparatus 102, according to some embodiments of the present invention. Auxiliary apparatus 102 includes a plurality of independent devices that may function in conjunction and communicate with substance sensor apparatus 100.

For example, an optional auxiliary apparatus 102 may include insulin pump 40, display 50 and controller 41. Insulin pump 40 may be any state of the art insulin pumps for example, the Paradigm 722 insulin pump from Minimed®, that is able to communicate with substance sensor apparatus 100 using any suitable communication protocol, for example wired, wireless, cellular, IR (infrared), RF (radiofrequency), Bluetooth, optical, or the like communication protocols. Auxiliary controller 41 includes switch/button 48 for manual operation and indicators 49 indicating when the controller is in use and for power supply status. Insulin pump 40, display 50, and controller 41 are connected, for example, using a pin lock assembly. As such, these components communicate according to any previously discussed protocol. Auxiliary apparatus 102 communicates with substance sensor apparatus 100 using any suitable communication protocol as described above.

Substance sensor apparatus 100 includes a treatment element 46, sensor 45, communication transceiver 47, and a wire 43. Substance sensor apparatus 100 is connected with controller 41 using wire 43, although wireless communication is also possible, for example with a transceiver at each of the apparatus 100 and controller 41 (not shown). Insulin pump 40 and display 50 communicate with substance sensor apparatus 100 by using communication transceiver 47, according to any conventional wireless communication protocols. It is worth noting, that a transceiver may comprise only a transmitter, a receiver, or both, either as an integral unit or separate components (according to some embodiments).

A treatment element 46 may be used for producing various treatments or stimulation, for example heating, modifying temperature, neural stimulation that induces vasodilatation, such as nociceptive axon reflex, massaging, mechanical vibration, acoustic vibration, ultrasound, suction, infusion or application of an additional substance or chemical to the skin and/or underlying tissue, applying a low electric field, applying a low magnetic field, light irradiation, radiofrequency ("RF") irradiation, microwave ("MW") irradiation, or the like treatment modality.

Substance sensor apparatus 100 includes a substance sensor (not shown) used to detect various parameters relating to a substance of interest, for example glucose, cholesterol, triglycerides, hemoglobin, white cell count, red cell count or the like substance or chemical. Substance sensor (not shown) is inserted subcutaneously.

Additional sensors 45 for example temperature sensors is coupled to substance sensor apparatus 100 to provide additional data to an associated controller that is for example an "on board" or an auxiliary unit.

For example, communication transceiver 47 may and form part of real time continuous glucose monitoring through implementation with the MiniLink REAL-Time transceiver which measures the glucose level continuously in the subcutaneous tissue and wirelessly sends (as indicated by arrows 44) the glucose ISF readings to insulin pump 40, which are displayed by display 50. The communicated ISF data may be used to depict the dosage of insulin to be infused by insulin pump 40.

Controller 41 obtains the substance sensor reading, for example glucose concentration, indirectly from insulin pump 40 or directly form the substance sensor transceiver 47. The received data are processed by controller 41 to depict and apply a treatment protocol using treatment element 46. Controller 41 controls the function of treatment element 46 using information received from secondary sensor 45 that is located near the treatment element 46.

According to some embodiments, treatment element 46 is provided in the form of a heating element including a plurality of layers (not shown). The plurality of layers include an upper layer that seals the element made of polyethylene; below that layer, there is an etched circuit, below which there is a metallic layer, such as copper layer, for heat distribution and mechanical support; below that layer, there is another sealing polyethylene layer, below which there is an adhesive biocompatible tape. For example, the heater may have a thickness of less than about 0.2 mm and diameter of about 3 cm, thin electric wires of length of about 60 cm, including small connectors at both ends to couple the heater to the controller unit 41. The power required for the heating element can be 2 Watts. The element is turned on and off by the controller 41 to stabilize the skin temperature in the range of from about 37°C to about 39°C, for a controllable length of time, for example 30 minutes, after which the temperature regulation may be stopped. In some embodiments, other heating temperatures, other durations or heating profiles of heating can be implemented as well as other heating powers can be used.

In some embodiments, the present invention is adapted for use in hospitalized patients. Many hospitalized patients are lying in bed most or all of the time so their local subcutaneous blood perfusion may be compromised. In those cases, local stimulation or treatment of the measured tissue region vicinity that improves the local blood perfusion can reduce significantly the substance delay as discussed above and improve the subcutaneous measurement accuracy. For example, such a sensor may improve the regulation of the glucose level of diabetic and non-diabetic hospitalized patients such as patients in intensive care units (ICU). Currently, because of the lack of accuracy of the subcutaneous or continuous glucose sensors, the blood glucose is measured in such patients either by pricking the patients for each measurement or drawing blood from arterial or venous line. The present invention provides, in some embodiments, a method to improve the accuracy of the continuous or subcutaneous glucose monitors by local treatment or stimulation of the vicinity of the tissue of interest that improves the local blood perfusion and/or reduces the variability of the glucose transport process from the blood till it measured by the sensor, as discussed above.

Reducing the delay of the glucose transport from the blood to the ISF and then to the sensor is important for better control of the glucose level since any delay in the glucose measurement induces errors in the glucose estimation and can thus cause errors in the treatment which may induce hypoglycemia or hyperglycemia. Currently, in hospitalized patients, such as ICU patients, glucose regulation is done by manually adjusting the blood infused insulin level according to a predetermined algorithm or protocol. In some embodiments, the insulin infusion rate is determined automatically by a processing unit that receives the glucose level and other parameters and sets the insulin infusion rate accordingly. In case of automatic insulin delivery for tight glucose level regulation, reducing and stabilizing the delay of the glucose transport from the blood to the ISF and then to the sensor, is very important or even critical. There are many attempts to compose such an "artificial pancreas" since the development of continuous glucose monitors. Currently, the blood insulin delivery and insulin dosing protocols are known in the art. The main obstacle for such an automatic tight glucose level regulation is an accurate continuous glucose sensor. Any delay such as the current delays of the glucose transport time and any variability in this delay induces an error for the control algorithm that will result in less tight glucose regulation. Thus, another use of the methods and devices described by the present invention is to combine them with a glucose sensor, insulin delivery device and a control algorithm to provide a better accuracy and robustness of a closed loop glucose level control system.

In some embodiments, such as in case of hospitalized patients, the substance sensor is connected to a bedside monitor, which may provide some or all of the functions of the auxiliary units mentioned before. For instance, the bed side monitor may display and/or log the levels of the measured substance. The bed side unit may control tissue treatment or stimulation or regulation to the vicinity of the tissue in which the substance is measured. For such a bedside unit, in addition to the exemplary treatments described herein, more power demanding treatments can be applied, such as strong massaging of the tissue, since the bedside unit may be connected to the main power line.

In some embodiments, for tight glucose level regulation the insulin is delivered directly to the blood system, such as through a venous line. The insulin can be delivered by an infusion pump or insulin pump connected to a venous line through which the insulin is delivered to the patient. The insulin delivery device is connected or controlled by the processing unit that gets the glucose readings from the improved subcutaneous glucose sensor with tissue treatment or regulation for the measured tissue region. Because of the improved accuracy of the glucose measurement the glucose regulation will be more accurate as well. In cases of longer periods between the glucose readings, the tissue treatment or stimulation or regulation can be applied only a short period before the measurement time, as discussed above.

In some embodiments, for tight glucose level regulation the insulin is delivered subcutaneously. Such cases can be either in hospitalized patients or outpatients or regular diabetic patients. In case of subcutaneous insulin delivery there is an additional delay to the glucose transport delays in the control loop which is the delay of the absorption of the insulin from the subcutaneous tissue to the blood and lymph system. U.S. Patent Application Serial No. 11/821,230, the disclosure of which is incorporated herein by reference in its entirety, discloses methods and devices to reduce the delay of the insulin absorption and to improve the repeatability of the insulin delivery. Combining those methods and devices with the methods and devices described in the present application for improving glucose sensing will provide a better accuracy for tight glucose level regulation.

In some embodiments, the same treatment or stimulation or regulation is applied to the vicinity of the tissue region in which the glucose level is measured and to the insulin infused tissue region vicinity. In some embodiments, the glucose is measured in a tissue region close to the insulin infused tissue region such that the same treatment or stimulation or regulation can be applied to both of them. In some embodiments, the glucose sensor is attached to the insulin infusion catheter, both are secured with the same securing element and a treatment or stimulation or regulation is applied to infused and measured tissue region vicinity. In some embodiments, the treatment profile can differ according to the performed action such as glucose measurement or insulin bolus infusion.

### EXAMPLE 1

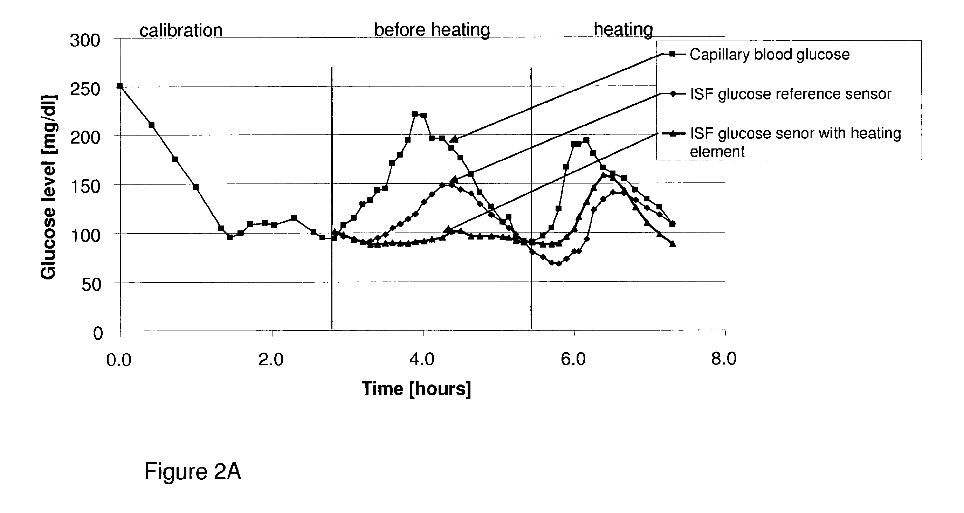

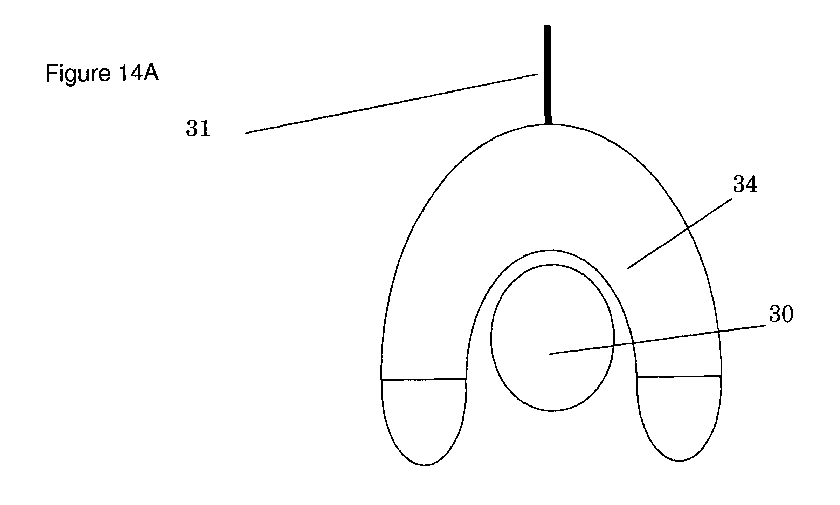

Figures 2A illustrates results obtained with the CGMS according to optional embodiments of the present invention with a diabetic patient. The treatment element used for these examples was a U shaped heater, as shown in more detail in Figure 14A, attached around a Guardian RT® glucose sensor by Minimed® measuring the ISF glucose level disposed at the waist. A second Guardian RT® glucose sensor was attached to the opposite waist as a reference ISF glucose measurement, not having a treatment element. The glucose levels were compared to capillary blood glucose level measured using finger pricking and glucose determination using an Elite® glucometer. The two sensors were in place for one day with two excursions of the glucose level between 100-250 mg/dl.

Figure 2A graphically illustrates the importance of using treatment element with the substance sensor according to the present invention to improved ISF glucose reading as a predictor of blood glucose concentration. A comparison of the effect of the treatment element as depicted with heat is shown comparing blood glucose levels to two substance sensor for ISF glucose levels. The square (■) curve represents the measured blood glucose levels that serve as the gold standard. The diamond (◆) point curve depicts the second ISF glucose sensor while the triangle (A) point curve depicts the first ISF glucose sensor having a treatment element, specifically a heating element, according to the present invention.

Initially a 2 hour calibration period was used following insertion of the ISF glucose sensors. During the second phase of the experiment glucose concentration was recorded from the three sensors for a 2 hour period. The results demonstrate that the ISF glucose sensors do not match the blood glucose measurements. The first ISF sensor diamond curve (◆) more closely depicted the blood glucose square curve (■) while the triangle curve (A) did not produce a comparable reading. Sensor reading during this second phase of the experiment demonstrates a known problem with state of the art ISF sensors, namely that their ability to predict the blood glucose levels is largely dependent on where they are inserted into the patient and additional unknown parameters; the diamond curve (◆) and triangle curve (A) are not similar in shape to each other, although the actual sensors are identical, therefore uniformity is an issue even in the same patient.

During the third phase of the experiment the treatment element, heat, was applied and sensor recordings recorded for a 2 hour period. Treatment in the form of heating was applied in the vicinity of the test sensor was heated to 39°C. As can be seen, the transport of the plasma glucose (square curve (■)) to the test sensor region (triangle curve (A)) was very slow before the treatment and was markedly improved after the heating treatment in the vicinity of the test sensor tissue region. In addition, it can be seen that the delay of the glucose readings in the test sensor with heat (triangle curve (▲)) relative to the blood glucose (square curve (■)) is significantly smaller (~10 minutes) than the delay of the reference sensor (diamond curve (◆) ), despite the initial disadvantage (without heat) of the test sensor as shown by the readings without heat.

### EXAMPLE 2

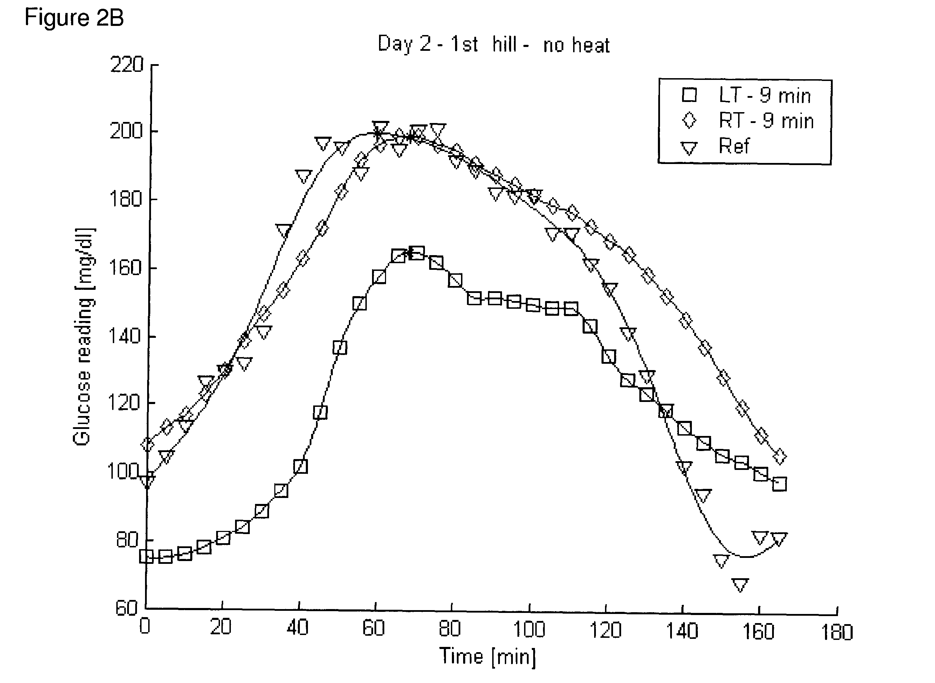

Figures 2B-2I show results of testing the CGMS including a treatment element according to some embodiments of the present invention in Type I diabetes. Figures 2B-2I illustrate the results of a test with a similar protocol performed with a different diabetic patient. Two MiniMed® ISF sensors, both having a heating treatment element, according to some embodiments of the present invention, were placed in the left hip (LT) and right hip (RT). The two sensors were compared to blood glucose levels (Ref) measured with a capillary blood stick test and used as the gold standard. The first day was used for calibration of the sensors, while testing was initiated on the second day; results are depicted during the second and third days following sensor placement. On the second day 2 glucose excursion "peaks" were detected, during which heating was applied to the right sensor (RT) vicinity only during the second peak. On the third day the heating was applied to vicinity of the left sensor (LT) during the first peak and to the vicinity of the right sensor (RT) during the second peak. Glucose peak delays were calculated by a function after low pass filtering of the reference data. The delay is depicted in Figures 2B-2E with an open circle (o) on each of the curves. The results of the 4 glucose excursions held in the experiments are shown in Fig. 2B-2E and in Table 1.

Table 1 shows the calculated delay between the blood glucose levels and the ISF glucose levels. The delay represented in minutes depicts the delays of the glucose peaks between MiniMed® sensors and reference blood glucose. A treatment element, heat, was used for the results shown in the cells marked with an asterisk (*).

**Table 1**

| | RT | LT | Improvement |
|---|---|---|---|
| | [min] | [min] | |
| Day 2 1 st peak (FIG 2B) | 9 | 9 | 0% |
| Day 2 2nd peak (FIG 2C) | 12* | 15 | 22% |
| | | | |
| Day 3 1st peak (FIG 2D) | 15 | 10* | 40% |
| Day 3 2nd peak (FIG 2E) | 7* | 14 | 67% |

Figure 2B depicts the three measured glucose levels during the first glucose excursion without treatment, providing a reference baseline in terms of measuring improvement. Although the delay is the same in both the left and right side when compared to blood glucose levels, it is clearly seen that the RT and LT curves do not resemble each other, despite the fact that the sensors themselves are identical and that they are inserted to the same patient (although on opposite sides of the body), showing the lack of accuracy and reliability of the sensor readings without treatment.

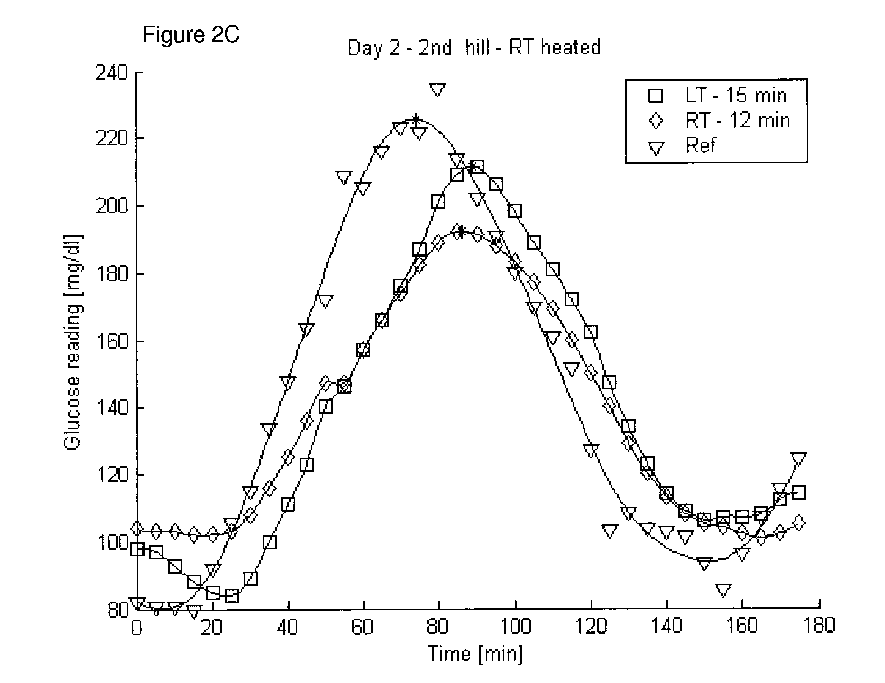

Figure 2C depicts the second glucose excursion undertaken during the second day, during which heating treatment was applied to the right sensor (RT). The RT curve more closely resembles the reference glucose curve following treatment according to the present invention. The applied treatment is attributed to a 22% improvement in peak glucose delay reduction shown between RT and the non treated LT sensor data.

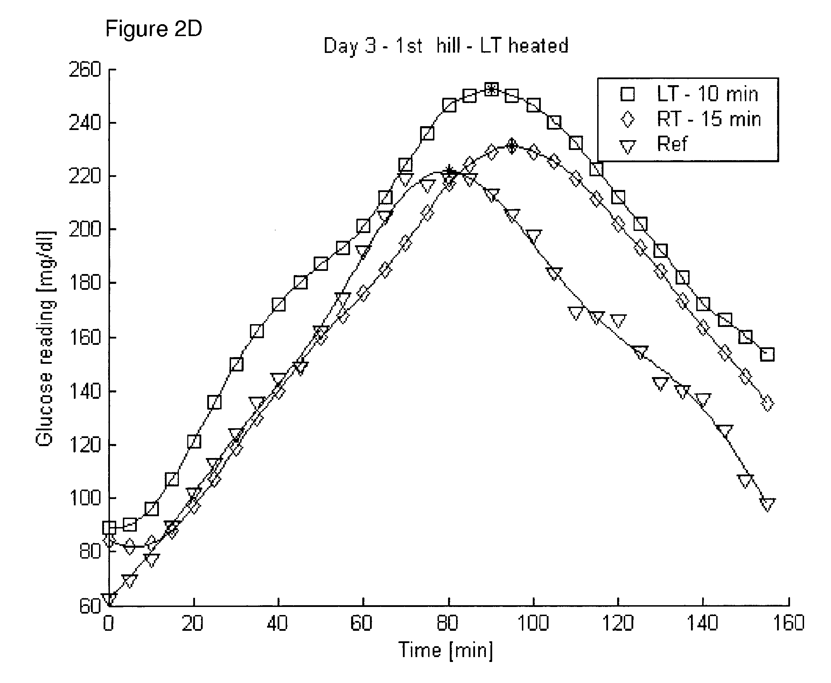

Figure 2D depicts the first glucose excursion during the third day during which heating treatment was applied to the left sensor (LT). The LT curve more closely resembles the reference glucose curve following treatment according to the present invention. The applied treatment causes a 40% improvement in the measurement of peak glucose levels when comparing the treated LT to the untreated RT sensors.

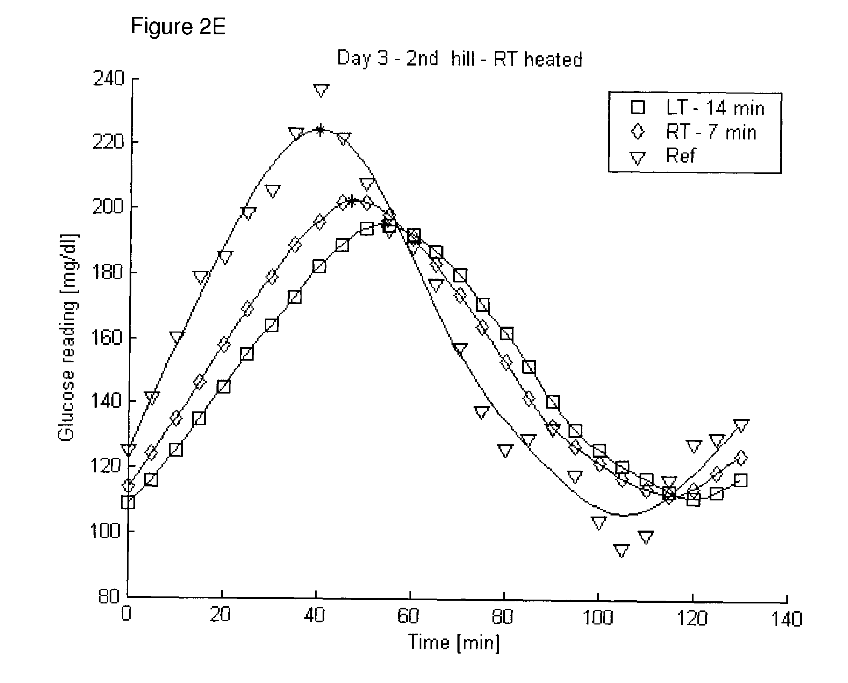

Figure 2E depicts the second glucose excursion during the third day during which heating treatment was applied to the right sensor (RT). The RT curve more closely resembles the reference glucose curve following treatment according to the present invention. The applied treatment causes a 67% improvement in the measurement of peak glucose levels when comparing the treated RT to the non treated LT sensors.

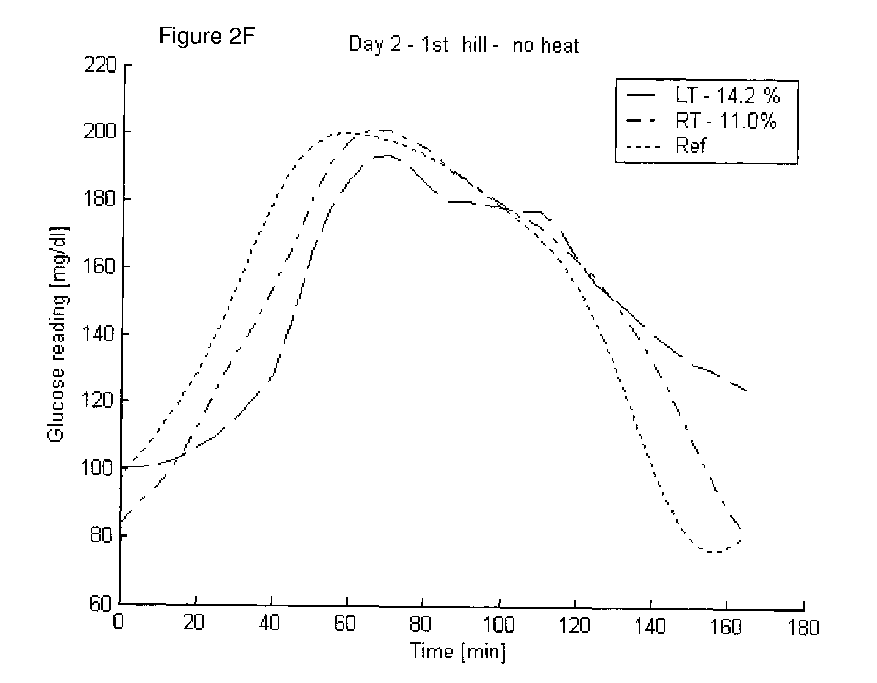

Figures 2F-2I illustrate results obtained with four glucose excursions of Figures 2B-2E, with the addition that measurements were followed by recalibration of the CGMS because the tissue conditions had changed. Recalibration is performed after treatment calibration of the sensors was performed under initial tissue conditions, without heat or other optional treatment. The use of a treatment, for example heat, improves or otherwise changes the transport coefficients, therefore the initial calibration and transport coefficients may no longer be valid, such that recalibration is performed to properly assess the transport coefficients under current tissue conditions. During recalibration, the accuracy of the glucose readings of the two ISF MiniMed® sensors placed in the left (LT) hip and in the right (RT) hip are compared to the reference blood glucose readings. The results obtained from the sensors in each of the four glucose excursions after recalibration were subjected to linear regression over the whole time range, as shown in each excursion graph, Figures 2B-2E, solving for ax+b while finding the optimal solution for the regression coefficients a and b respectively. The results after recalibration are summarized in Table 2 in terms of Mean Absolute Relative Difference (MARD) before and after recalibration.

**Table 2**

| | RT | LT | Improvement |
|---|---|---|---|
| | MARD [%} | MARD [%} | |
| Before Recalibration | | | |
| Day 2 1st peak (FIG 2F) | 14.2 | 23.6 | 50% |
| Day 2 2nd peak(FIG 2G) | 14.9* | 16.4 | 10% |
| | | | |
| Day 3 1st peak (FIG 2H) | 14.2 | 23.8* | -50% |
| Day 3 2nd peak (FIG 2I) | 11.2* | 15.8 | 34% |
| | | | |
| After Recalibration | | | |
| Day 2 1st peak (FIG 2F) | 11.0 | 19.1 | 54% |
| Day 2 2nd peak (FIG 2G) | 14.1* | 17.3 | 20% |
| | | | |
| Day 3 1st peak (FIG 2H) | 12.5 | 7.9* | 45% |
| Day 3 2nd peak (FIG 2I) | 11.5* | 16.2 | 34% |

Figure 2F depicts results of the first glucose excursion on day 2 without any applied tissue treatment. The three measured glucose levels during the first glucose excursion in the initial phase are illustrated in Figure 2B. The recalibrated results show improved performance, Figure 2F, when compared to the initial day 2 measurement, Figure 2B, both without treatment.

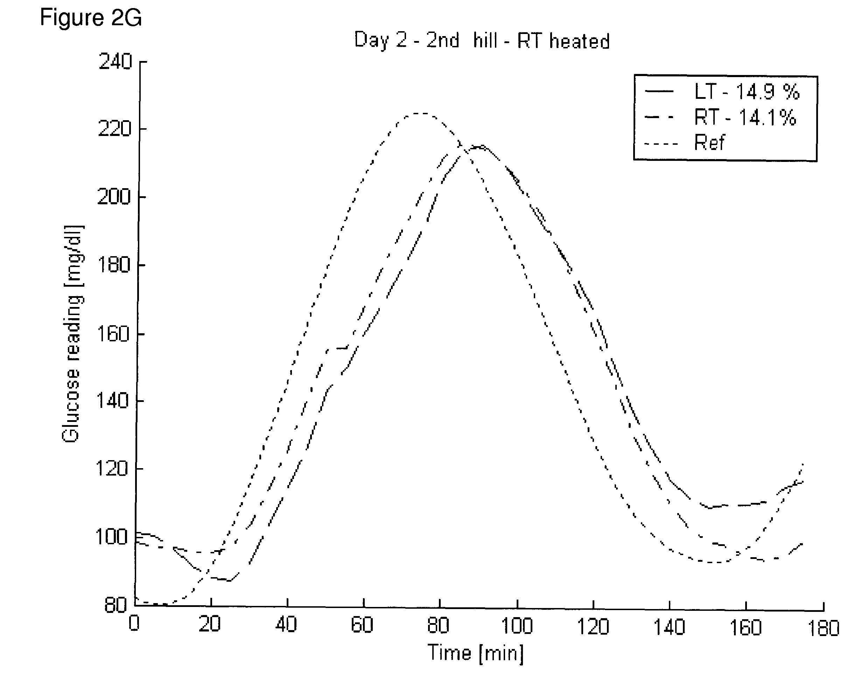

Figure 2G depicts results of the second glucose excursion on day 2 with tissue treatment on the right (RT) sensor, showing 20% better performance of the heated sensor with recalibration of Figure 2C.

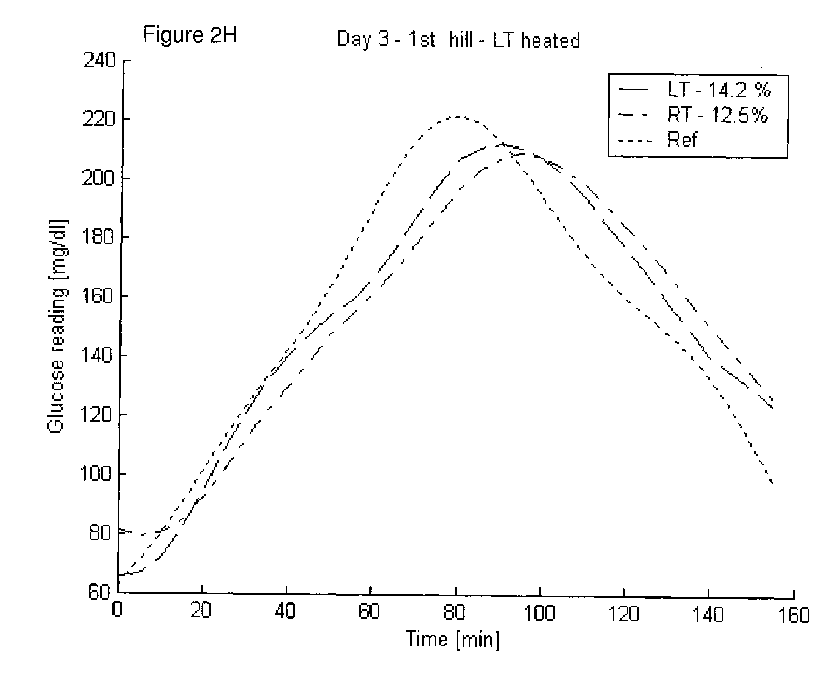

Figure 2H depicts results of the first glucose excursion on day 3 with tissue treatment on the left (LT) sensor, showing 45% better performance of the heated sensor of the results depicted in Figure 2D, following recalibration the CGMS according of Figure 2H.

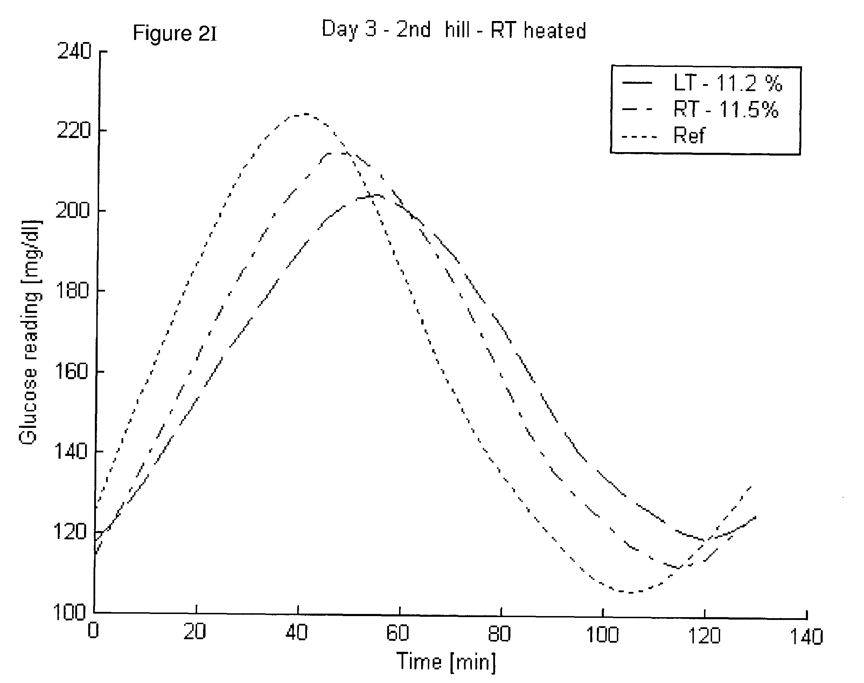

Figure 2I depicts results of the second glucose excursion on day 3 with tissue treatment on the right (RT) sensor, showing 34% better performance of the heated sensor with recalibration of Figure 2E.

The conclusions of the last test results are that the delays of the heated sensors, as summarized by Table 1, are consistently shorter and that the accuracies of the heated sensors after the linear recalibration were consistently better than the non heated sensors, as shown in Table 2. Also it can be seen that the MARD of the heated sensors (right sensor in both cases) during the second peaks was better after recalibration. The MARD of the 1^{st} peak of the 3^{rd} day was useful before the recalibration but was much improved (MARD of 7%) after the linear recalibration. Therefore both tests demonstrate that without the treatment, larger variability of the performance or accuracies of the sensors is obtained, while after the treatment and proper calibration for the heated sensor, reduced variability of the sensor accuracy is obtained.

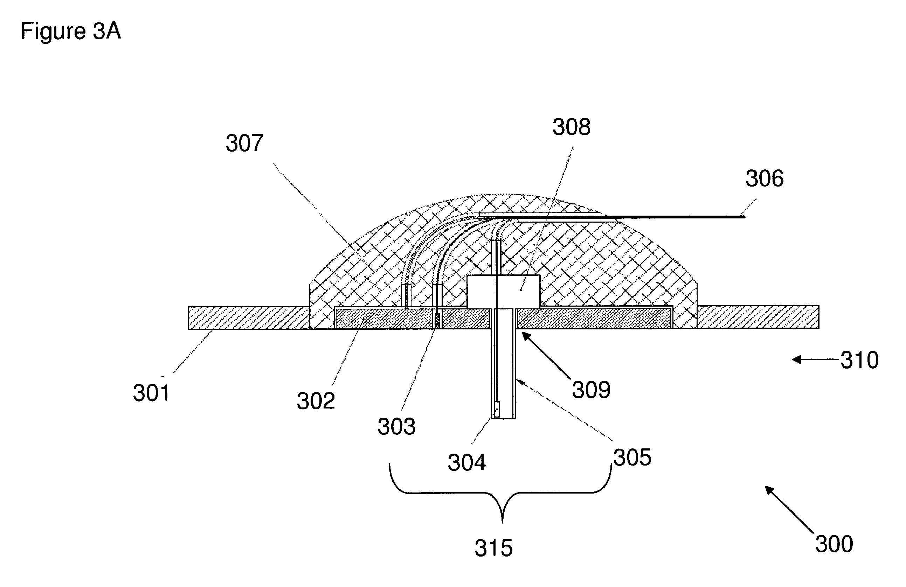

Figures 3A-C depict optional embodiments of the substance sensor apparatus 100 having a treatment element as depicted in Figure 1. As shown in Figure 3A, in some embodiments, the substance sensor apparatus 300 includes a heating treatment element 302 that adheres to the skin 301 around the substance sensor insertion point 309. Device cover 307 may be a flat circular structure including an opening in its center that defines insertion point 309 for the substance sensor 305 that penetrates skin surface 301 into the subcutaneous tissue 310. Substance sensor 305 is a catheter, as schematically illustrated. The reaction or activity required for substance sensing by sensor 305 may be performed within sensor 305 within the subcutaneous tissue 310 or outside on the skin surface in a separate compartment 308. Substance sensor 305 extends to compartment 308 which includes a controller or circuitry required for the sensor apparatus 300. Compartment 308 may include but is not limited to electronic circuitry required to operate substance sensor 305 as in the case of enzyme based glucose sensors for example. Compartment 308 may include circuitry required to perform signal modulation, conditioning, amplification, sampling, or communication with other auxiliary units, such as that depicted in Figure 1. Compartment 308 may communicate with one or more external auxiliary units (not shown) using wired or wireless communication protocol for example cellular, IR, RF, optical, Bluetooth or the like communication protocols.

According to some embodiments, treatment element 302 is not in contact with substance sensor 305, to avoid affecting measurements by the activity of treatment element 302 that may include heat. Protecting substance sensor 305 for example from overheating may be accomplished via device cover 307 that is and made of thermally isolating material, more applied so as to protect the user as well as substance sensor 305 from any deleterious effects of the activity of treatment element 302.

Control of the treatment protocol is governed by a controller in an auxiliary unit (not shown) or alternatively and in compartment 308. The treatment profile is controlled using a controller (not shown) that analyzes the sensed data. The controller controls and receives data from treatment element 302, secondary sensor 303, and substance sensor 305. Controller may control any aspects relating to the treatment protocol, its parameters, activity, inactivity or the like.

Substance sensor apparatus 300 may further include one or more additional sensors 304, in the form of a temperature sensor. Additional sensor 304 is located within or adjacent to substance sensor 305, within the measured tissue region 315. Additional sensor 304 provides better control of the characteristics associated with the substance measurement tissue region 315, for example temperature. Specifically, allowing substance sensor apparatus 300 via a controller to regulate the temperature inside the measured region 315 to a fixed optimal temperature, providing better stabilization of the substance transport and measurement process can be achieved. The local temperature variations in the measured region induced by ambient temperature variations as well as other factors induce variations in the blood perfusion and facilitate larger variability of the substance transport and measurement process that results in adding delays or errors to the substance measurement.

In some embodiments, the heating element 302, and one or two (or more) of the optional temperature sensors 303 and 304 are connected to an auxiliary unit (not shown) using cable 306. The auxiliary unit may include the power source, controller, secondary treatment element, drug delivery device, display or the like.

Substance sensor apparatus 300 may be attached to the skin layer 301 using an adhesive layer (not shown). The adhesive layer 301 can also cover the treatment element 302 (not shown). The adhesive layer 301 may be a thermal conducting adhesive or a thin adhesive layer, a laminate covered adhesive that is peeled off by the user before insertion of the substance sensor 305 and attachment of the treatment element 302.

Substance sensing apparatus 300 further includes a flexible catheter, for the insertion of the substance sensor 305, which is placed within the subcutaneous tissue 310 in the tissue treatment area 315 using a sterile needle inside the catheter (not shown) that is pulled out after insertion of the catheter to the required tissue region 315.

The treatment element 302 may be provided with a thermally conducting adhesive layer (not shown) that is in contact with the skin layer 301, an electrically isolating layer (not shown) with temperature sensors, a heating layer, a thermally isolating layer and an adhesive layer for attaching heating device 302 to additional thermal isolation provided by device cover 307 if needed. All layers can be manufactured using printing techniques and mass production methods.

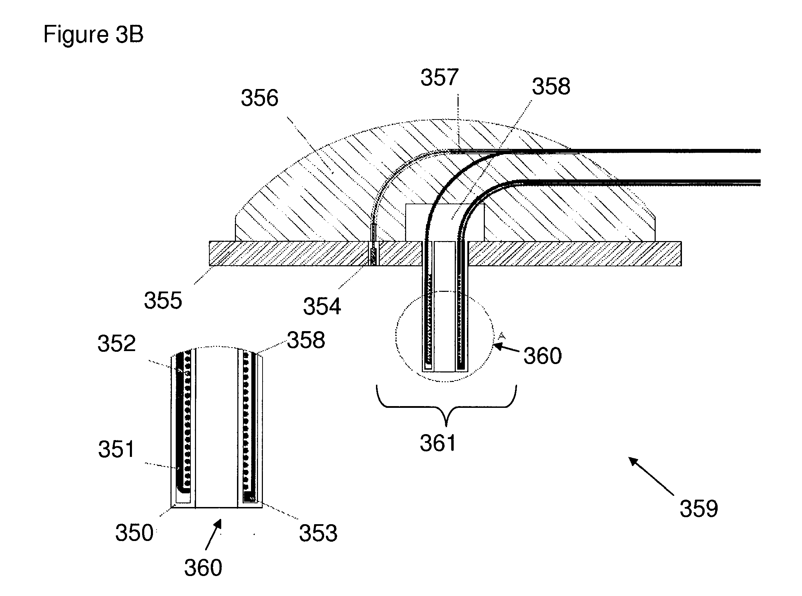

An optional device for heating the measured tissue region is illustrated in Figure 3B, wherein the treatment element is placed with the substance sensor. The substance sensor 360 includes a heating element 352 along its distal portion 350 that is adjacent to the measured tissue region 361. Treatment element 352, in the form of a heater, may be made of a conductive wire or material with high enough resistance and good strength and durability. The conductive wire or conductive material may be comprised of tungsten wires, deposition of thin copper strip, or the like. Heating element 352 may be embedded into the substance sensor tube 360 during its manufacture, using methods known in the art. This can be done by wrapping the wire coil on a thin wall tube and then covering it with a second polymeric layer. The opposite side of the heating wire coil 351 is placed within the tube as well. In some embodiments, the heating wire can be shaped in other forms such as a single loop or zigzag or in another optimal form that can be efficiently manufactured to provide the required heat for the measured tissue region. An advantage of heating within the tissue is a smaller volume of tissue around the measured region is heated and hence requires less electric power. Also, the temperature of the heated volume, usually in the subcutaneous tissue, may be more easily regulated since it is more isolated from the skin temperature which may be different from the ambient temperature.

In some embodiments, a plurality of sensors may be incorporated into apparatus 359. Sensor 353 may be placed inside the catheter tube 360, while monitoring the measured tissue region 361. Temperature sensor 353 provides better control of the temperature of the measured tissue region 361.

In some embodiments, the controller (not shown) can be contained in the treatment device for example in compartment 358 or in an auxiliary unit (not shown). In some embodiments, the controller controls the treatment element 352 and the treatment protocol in accordance with data received from the plurality of sensors, 354 and 353.

In some embodiments, device cover 356 provides support for the catheter attachment to the body and provides thermal isolation that further reduce the power requirement and consumption of the treatment element 352. The heating device 352 is attached to the skin layer with an adhesive layer 355. The adhesive layer 355 may be covered with a laminate (not shown) that is peeled off by the user before insertion of the substance sensor 360 and attachment of the heating device 352.

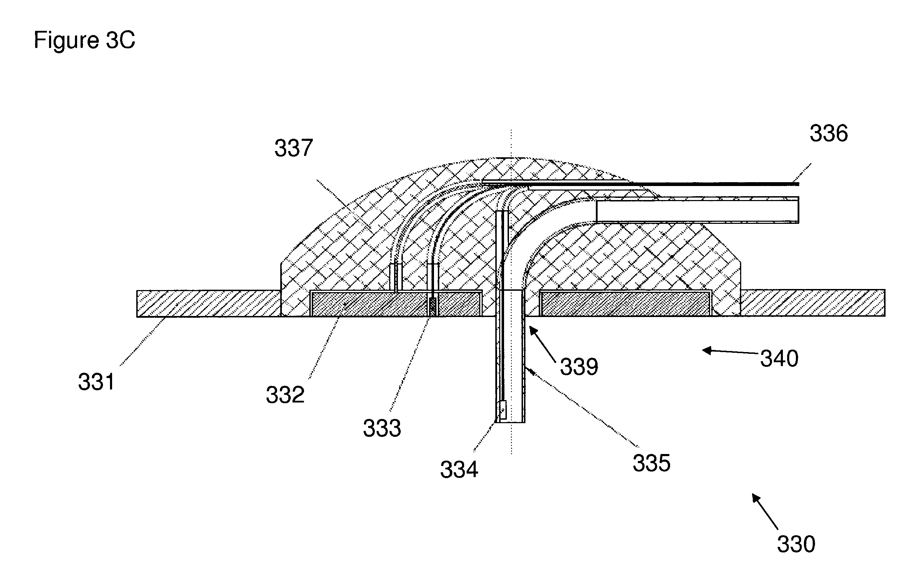

As shown in Figure 3C, in some embodiments of the present invention, the substance sensor apparatus 330, sensing occurs outside of the subcutaneous tissue 340 using one or more microdialysis substance sensors 335, coupled to a fluid pump 334 that transports the sample to the sensing portion. Microdialysis substance sensor 335 may be connected to a larger auxiliary sensing unit (not shown) that includes the fluid reservoirs and/or the pump 334. Microdialysis substance sensor 335 may include a catheter extending out of the treatment apparatus 337 and connected to an auxiliary sensing unit (not shown) that handles the fluid flow and/or the substance concentration measurement in those fluids. An apparatus 330 includes a treatment element 332, in the form of a heating element (not shown) a printed circuit board (PCB) having the heating elements. Treatment element 332, in the form of a printed circuit board, includes a temperature sensor 333. A cooling element (not shown) may be included if more demanding temperature profiles are used.

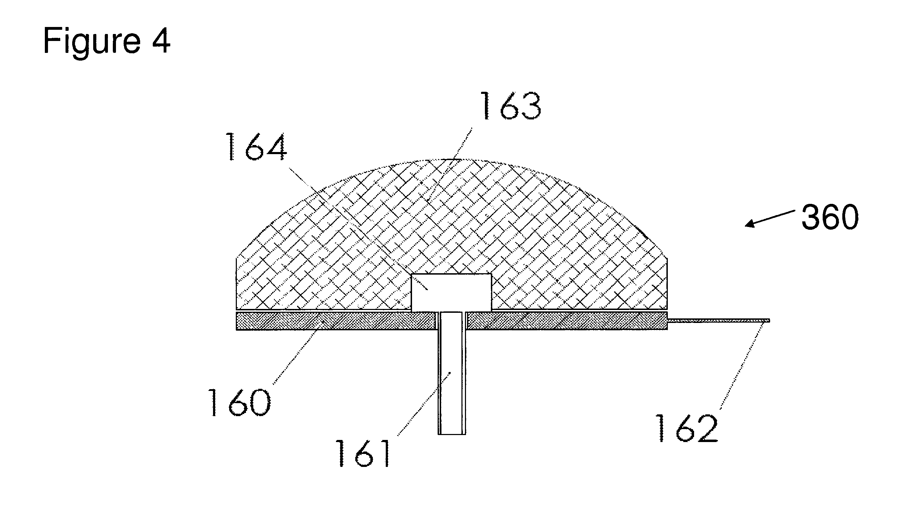

Figure 4 depicts an exemplary substance sensing apparatus 360 that may be comprised of disposable and reusable portions, according to some embodiments of the present invention. The disposable portions include device cover 163, substance sensor 161, and compartment 164. In some embodiments, treatment element 160, which is a heating element, is reusable. Treatment element 160 is shaped as a thin disk that is inserted between the disposable device cover 163 including substance sensor 161 and compartment 164. Treatment element 160 may further include a sensor such as a temperature sensor (not shown) used to control the treatment protocol. The temperature sensor can be part of a thermostat that automatically regulates the heating temperature by connecting and disconnecting the heater element power lines, or other self regulating heaters, such as PTC thermistors, and/or increasing or decreasing the power supplied to the heater.

In some embodiments, prior to attaching the device to the skin and penetrating the subcutaneous tissue, reusable treatment element 160 may be adhered or attached to the disposable portion 163 such that the treatment element 160 is in contact with the skin above the measured tissue region. In some embodiments, disposable and reusable portions may be coupled using a special mechanical connector or jig. Treatment element 160 may be manufactured to fit a plurality of substance sensors. Both treatment element and device may be comprised of disposable material.

Power may be provided to the substance sensing apparatus 360 using an auxiliary device that is connected via wire 162 to the treatment element 160. The reusable treatment element 160 may perform one or more of the treatments or types of stimulation discussed herein, heating, massaging, vibrating, acoustic treatment or stimulation, optical radiation, RF radiation, MW radiation, applying electrical field etc.

In some embodiments, device cover 163 may be made wider than reusable part 160 such that the rims of the disposable part are used for attaching or securing the treatment device to the skin.

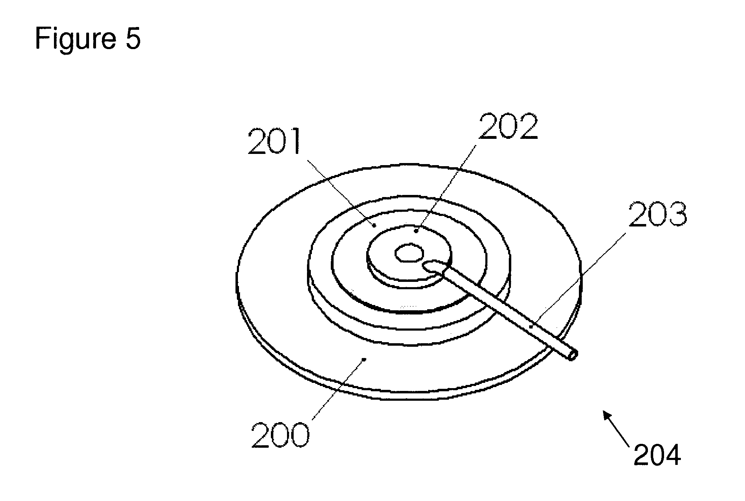

Figure 5 depicts an embodiment of the present invention including a tissue treatment device 204 that vibrates a treated tissue region in which the substance is measured. Treatment device 204 includes electric motor 202, rotating disk 201 with asymmetric load, wherein electric motor 202 and rotating disk 201 together form a vibrating element. When in motion rotating disk 201 causes the treatment device 204 to vibrate in a circular vibratory motion. Treatment device 204 is coupled to the skin with an adhesive layer 200 where treatment device 204 vibrates the tissue underneath the treatment device 204 and the substance sensor tip (not shown). Treatment device 204 uses vibrating motion parameters commonly used in tissue massaging applications know and accepted in the art, for example frequency of from about 1 to about 50Hz and motor velocity of from about 60 to about 300 rpm.

In some embodiments, element 204 further includes cable 203 that connects the motor 202 to an auxiliary unit (not shown), for example, a power supply. As can be understood by one skilled in the art, other frequencies or rotational velocities can be used as well. The motor axis can be horizontal with the rotating disk 201 vertical to the skin surface. In this case, the vibrations are vertical to the skin surface in addition to horizontal.

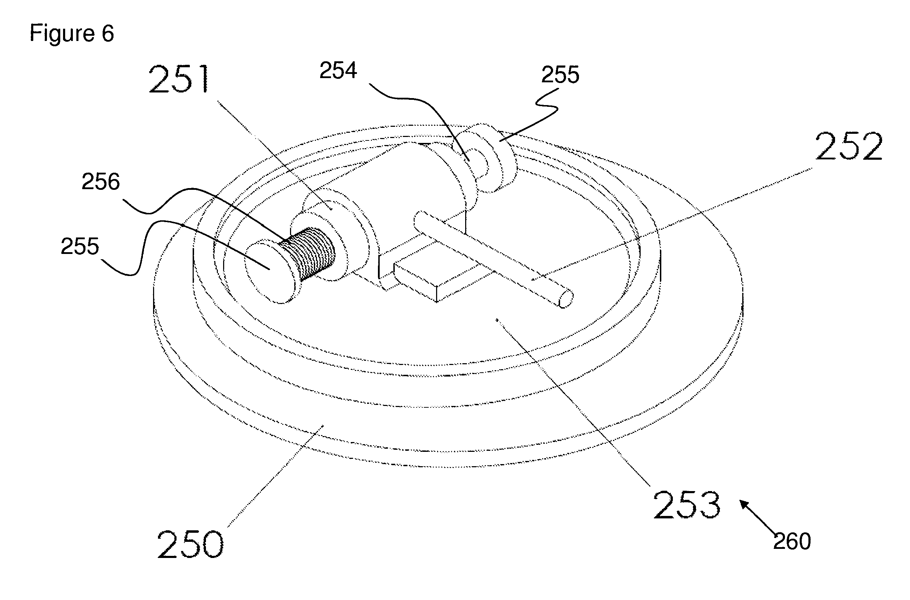

As shown in Figure 6, in some embodiments, a tissue treatment element 260 is provided that vibrates a treated tissue region in which the substance is measured. Treatment element 260 includes an electromagnet 251 that pulls a ferromagnetic rod 254 with two weights 255 at opposite ends thereof. A spring 256 returns ferromagnetic rod 254 to the initial location once the electromagnet 251 is turned off.

In some embodiments, a controller (not shown) may provide a periodical signal to the electromagnet 251 causing the rod 254 and weights 255 to vibrate at the periodic signal frequency, in turn inducing vibrations to the treated tissue underneath. To improve vibration efficiency, the rod 254, weights 255 (according to mass) and the spring 256 (according to force) can be designed to have a mechanical resonance frequency at the required frequency for massaging the measured tissue region. Cable 252 connects the motor to an auxiliary unit that functions as a power supply to treatment element 260.

Treatment element 260 may be provided with a resonance frequency that, upon being applied to the electromagnet 251, vibrations of larger amplitude are induced. Adhering the treatment element 260 to the skin with an adhesive layer 250 allows the treatment element 260 to vibrate the tissue underneath the treatment device and the substance sensor.

In some embodiments, the vibration axis can be designed to vibrate to other directions, such as vertical or perpendicular to the skin surface. In some embodiments, the vibration device can vibrate mainly the substance sensor either horizontally or vertically using vibration mechanisms that induce mechanical stimulation of the tissue and/or the neural response near the substance sensor. The vibrations can also modify the process of the Foreign Body Response (FBR) of the tissue that covers the substance sensor with a biofilm that slows the transport of the substance molecule into the sensor and changes the substance sensor's calibration.

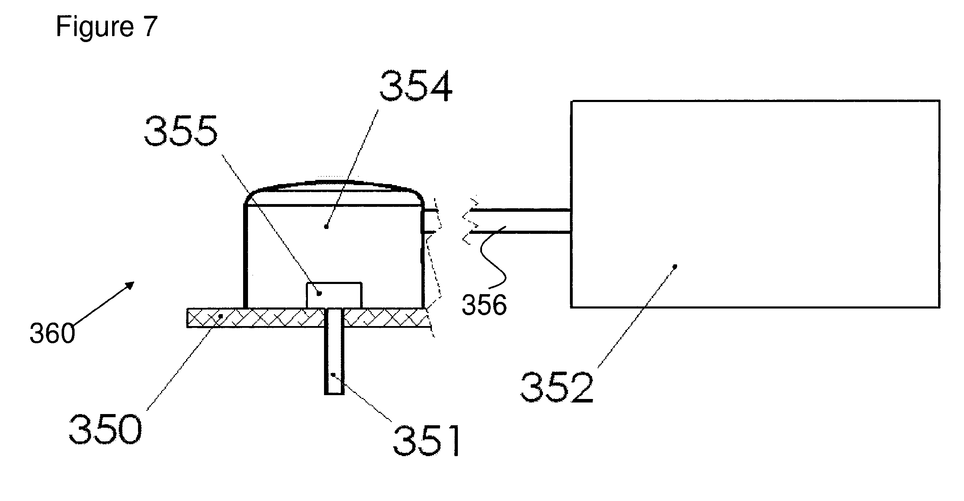

Figure 7 depicts a treatment device 360 that includes a massaging treatment element 354 that is comparable to the vibrating treatment element depicted in Figures 5 and 6, but with lower frequency and larger amplitude. Treatment device 360 is a single use or disposable item including substance sensor 351, connected to compartment 355 inserted into the subcutaneous tissue, located in the middle of a chamber 354 having a rigid wall, except at the side directed toward the skin, and flexible membrane 350. The flexible membrane 350 is adhered to the skin with adhesive layer (not shown). Chamber 354 may be connected with a tube 356 to an auxiliary unit 352 that provides compressed air to chamber 354. Treatment device 360 performs tissue massaging according to a treatment protocol by pumping air in and out of chamber 354 through a tube 356 via an auxiliary pump unit (not shown) in auxiliary unit 352. Control of the treatment protocol is accomplished by controlling the functioning and parameters of auxiliary unit 352 while the disposable portion of the unit can be relatively simple and low cost.

In some embodiments, when the air is pumped out of chamber 354, flexible membrane 350 curves in the upward direction into the chamber 354 pulling the tissue adhered thereto. Similarly, when the air is pumped into the chamber, the flexible membrane 350 curves in the inward direction away from chamber 354 and pushes the tissue adhered thereto.

In some embodiments, the massaging process is done periodically according to a typical frequency as is known and accepted in the art at about 0.01-10Hz, or the like frequencies. A massaging motion may be obtained by using a medium other than air or a gas, for example a liquid, such as water, oil or the like. For example, chamber 354 may be filled with an incompressible fluid, such as water, and appropriate auxiliary pump causes the fluid to flow in and out bringing about a movement in membrane 350.

Vibrating membrane 350 may be comprised of a rigid surface having a plurality of openings that are covered with a flexible membrane over the openings to improve adhesion to the skin and to spatially modulate the skin. The surface of vibrating membrane 350 that is in contact with the skin includes small features or bumps that extend out of the surface to contact the skin to improve massaging effect to the tissue. Vibrations or massage treatment protocols also prevent or slow the FBR (foreign body recognition) process of the tissue.

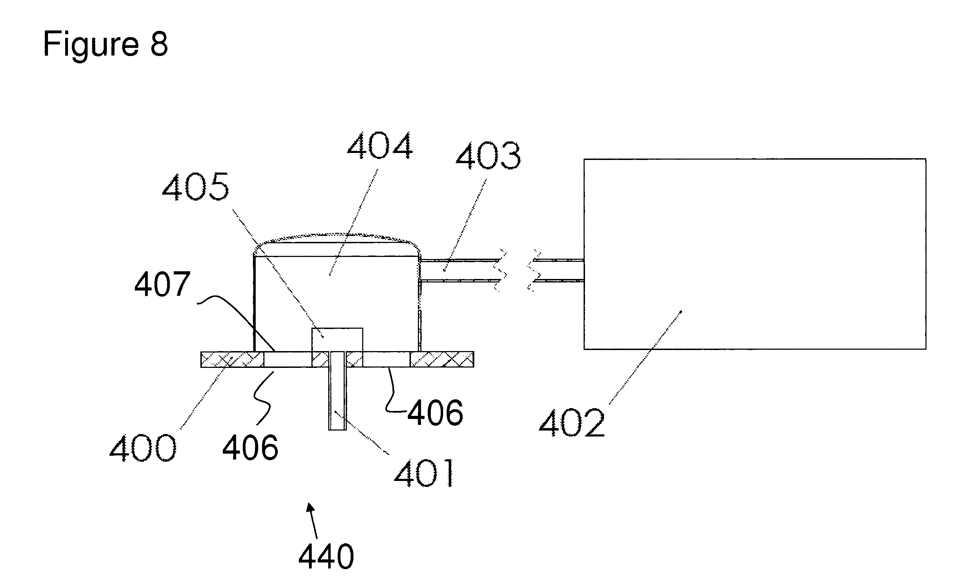

Figure 8 depicts an additional treatment device 440 that provides suction over a treatment area in the vicinity of the substance sensor. Tissue suction is known to improve blood perfusion in that tissue region. Treatment device 440 is and disposable including a substance sensor 401 connected to compartment 405 including circuitry and a power supply, required for substance sensing. Substance sensor 401 is located in the middle of a chamber 404 having rigid wall all around except of the skin side. The walls of chamber 404 are adhered to the skin with a circular adhesive layer 400 having a plurality of openings 406 that seals the chamber rim to the skin. The adhesive layer 400 is attached to the skin during the substance sensor insertion process to secure the substance sensor 401 in its position. Chamber 404 is connected to an auxiliary unit 402 via tube 403. Auxiliary unit 402 is an air pump, liquid pump or power supply or the like used to crate suction. One of skill in the art will appreciate that some embodiments of the present invention may include a pressurization treatment element, that pressurizes the tissue area at issue.

Skin suction may be accomplished by pumping air out of chamber 404 through tube 403 via a pump provided in auxiliary unit 402. The control of the treatment protocol is accomplished by the auxiliary unit 402 and the disposable unit 440 can be made simple and low cost.

In some embodiments, suction is accomplished according to a predetermined treatment protocol. For example, suctioning every 10 minute can be applied. Another example is applying a vacuum in chamber 404 for 30 seconds and then releasing the vacuum for additional 30 seconds. This process can be repeated several times in order to increase blood perfusion in the tissue region underneath the treatment device. In some embodiments, the chamber opening to the tissue can be made of a rigid surface with a plurality of openings 406 to increase adhesion area to the skin and to spatially modulate the skin suction. The repeated tissue suction can also prevent or slow the FBR process of the tissue.

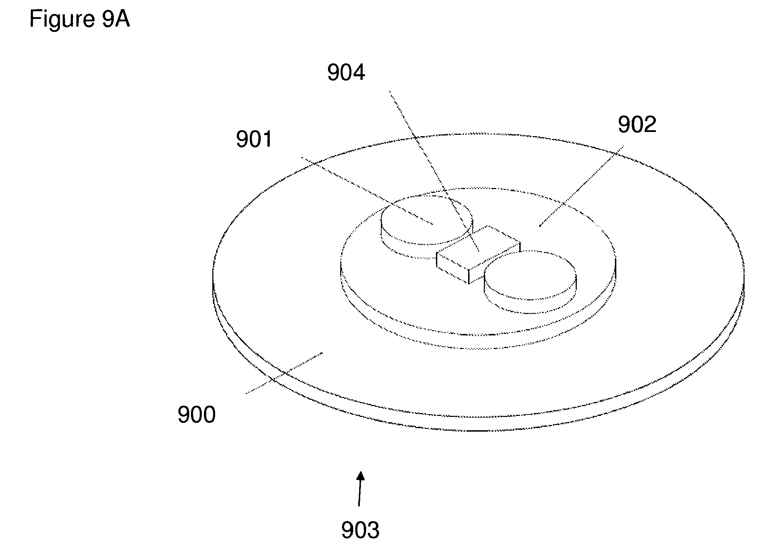

Figure 9A-B depict optional embodiments of the substance sensor according to the present invention including a tissue treatment device that uses optical radiation to stimulate the tissue region. Figure 9A depicts an upper view of treatment element 903 including at least one or more optical radiation element 901 attached to the skin using adhesive layer 900 around the substance sensor (not shown) that is centered about treatment disk 902 having a central opening for the substance sensor that enters the subcutaneous tissue. Compartment 904 includes circuitry relating to the treatment element, substance sensor (not shown) taking the form of a power supply, controller or the like.

Optical radiation element 901 can be made of various optical radiation sources for example LEDs, laser diodes, lamps, or the like as known and accepted in the art. The optical radiation energy may be in the visible, NIR and MIR regions. The light source may emit pulsed light or CW light and the pulsed light source may further emit pulses that are appropriate to generate photoacoustic or thermoacoustic signals on the substance sensor and/or in the tissue region close to the substance sensor. The optical treatment or stimulation can also modify the FBR process of the tissue.

The adhesive layer 900 can be provided on the outer ring area of 900 or cover the optical radiation element 901 with an optically transparent adhesive, that is transparent in the treatment protocol's relevant optical wavelength range. The adhesive layer is covered with a laminate (not shown) that is peeled off by the user before insertion of the substance sensor (not shown) and attachment of the optical radiation device.

The light source may be disposed in an auxiliary unit (not shown) and delivered with at least one or more optical fiber to the optical radiation treatment device. The optical radiation source is connected to an auxiliary unit using an electrical cable (not shown) or to electronics disposed as part of the optical radiation treatment device.

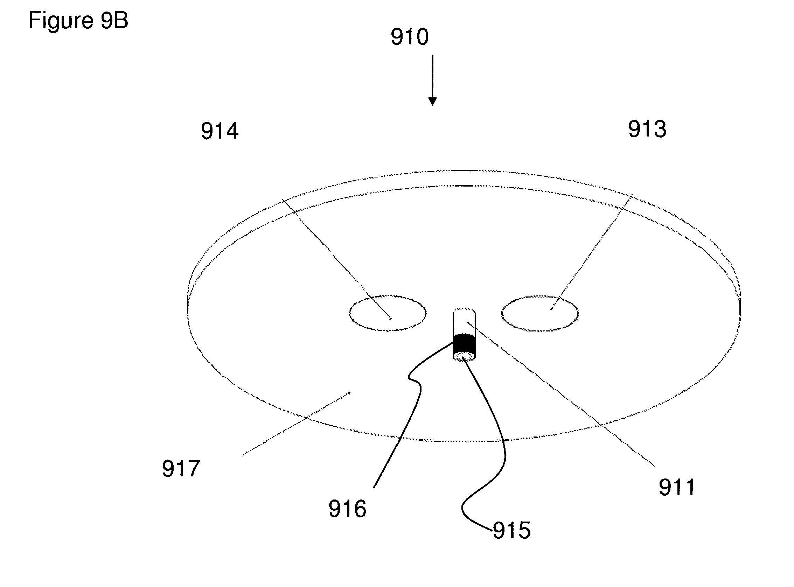

A further depiction of an optical treatment element as depicted in Figure 9A is introduced in Figure 9B. Figure 9B depicts an underside view of an optional optical substance sensor 910. Substance sensor 915 is coated at its tip 916 with an optical absorption coating 911. Coating 911 functions to absorb the wavelength or some of the wavelengths produced by the optical radiation element 914 and 913. Optical irradiation elements 913 and 914 may include one or more light sources as known and accepted in the art for example LEDs, laser diodes, lamps, or the like. The light source may emit pulsed light or CW (continuous wave) light and the pulsed light source may further emit pulses that are appropriate to generate photoacoustic or thermoacoustic signals on the substance sensor tip 916.

The optical irradiation wavelength can be either in the visible region or in the NIR (near infra-red). In some embodiments, using wavelengths in the range of 700-1000nm provides relatively low absorption of the optical radiation in the tissue. Consequently, a larger portion of the illuminated radiation can be scattered in the tissue and absorbed in the substance sensor tip. The tip-absorbed optical radiation can induce a local hit around the substance sensor tip and efficiently heats the measured tissue region. Using shorter wavelengths in the visible region, but also in the 700-1000nm region, can increase the portion of the radiation absorbed by the hemoglobin and consequently can heat more blood or hemoglobin reach regions in the irradiated tissue region. Using longer wavelengths in the NIR, MIR (mid-infrared) or FIR (far infra-red) regions can increase the portion of the radiation absorbed by the water in the tissue and consequently can heat more of the water to reach regions in the irradiated tissue region. Also, in case of using light pulses to create photoacoustic treatment or stimulation, the portion of treatment or stimulation induced at the substance sensor tip, hemoglobin regions or water regions, such treatment or stimulation can be according to the absorbed radiation distribution and the photoacoustic coefficient of each region. The produced photoacoustic signal can be measured using an acoustic sensor disposed skin attachment structure 917 and can be used for monitoring the energy absorbed in each of those regions or substance sensor tip 911. An auxiliary unit may contain the acoustic sensor (not shown).

In some embodiments, some of the wavelengths of the above mentioned regions can be used for better control of the heated or stimulated region of interest. In some embodiments, at least one of the wavelengths is absorbed by a substance sensor tip coating and at least one wavelength is not absorbed by the coating to better control of the heated or stimulated region. The algorithm to control tissue treatment or stimulation can obtain information from tissue temperature sensors (disclosed above), acoustic sensor, optical sensor, and additional tissue parameters. The algorithm can control wavelengths to regulate the substance kinetics from the blood system.

A device similar to the one illustrated in Figures 9A-B can irradiate the measured tissue region, externally or internally, respectively, with radio frequency (RF) radiation or microwave (MW) radiation. Another optional embodiment can apply an electric field to the measured tissue region using, for instance, 2 electrodes similar to optical radiation elements 901 shown in Figure 9A, to apply the field to the skin or using electrodes disposed on the external side of the substance sensor tip inserted into the tissue. Also, the same device can be used to apply high or low frequency fields and even a DC (direct current) field. To improve the electrical contact the adhesive layer can be a conducting hydrogel or other known in the art materials to attach electrodes. The treatment or stimulation can also prevent or slow the FBR process of the tissue.

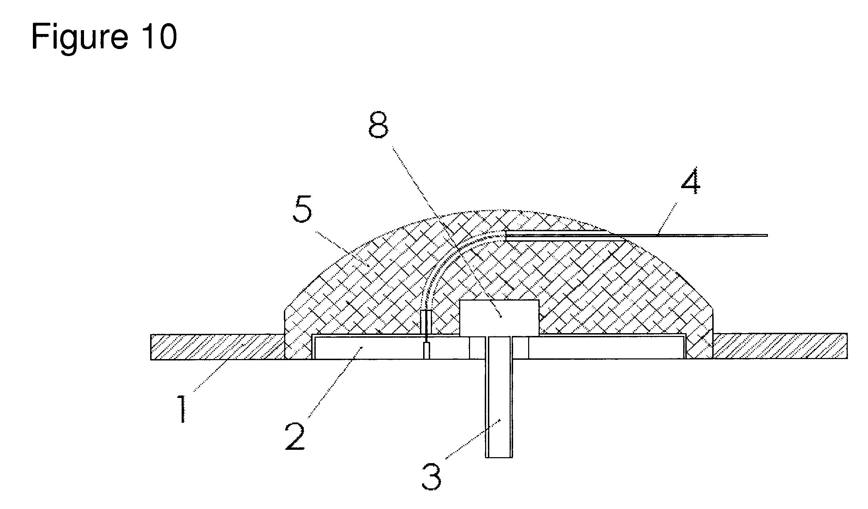

As shown in Figure 10, a substance sensor having an acoustic stimulation treatment element according to some embodiments is provided to improve the sensor functionality. The substance sensor 3 is combined with an acoustic stimulation element 2 that is coupled to the skin around the substance sensor 3. Device cover 5 includes a central opening allowing substance sensor 3 to enter the subcutaneous tissue. Acoustic stimulation element 2 may be made of piezoelectric materials for example PZT or PVDF, or the like. Acoustic stimulation used by the treatment device may include low or high acoustical frequencies or higher frequencies in the ultrasonic region. The acoustic treatment or stimulation can also modify the FBR process of the tissue.

The acoustic treatment or stimulation device is attached to the tissue with an adhesive layer 1. Adhesive layer I may have variable width covering the acoustic treatment or stimulation element with an acoustic conducting adhesive, for example adhesive hydrogels. The acoustic treatment cover 5 or stimulation element 2 may be covered with an acoustic conducting layer for example acoustic hydrogel or liquid.

Acoustic treatment or stimulation element can be either connected to an auxiliary unit (not shown) using cable 4. An auxiliary unit may include but is not limited to a further treatment element, power supply, and/or an acoustic treatment device.

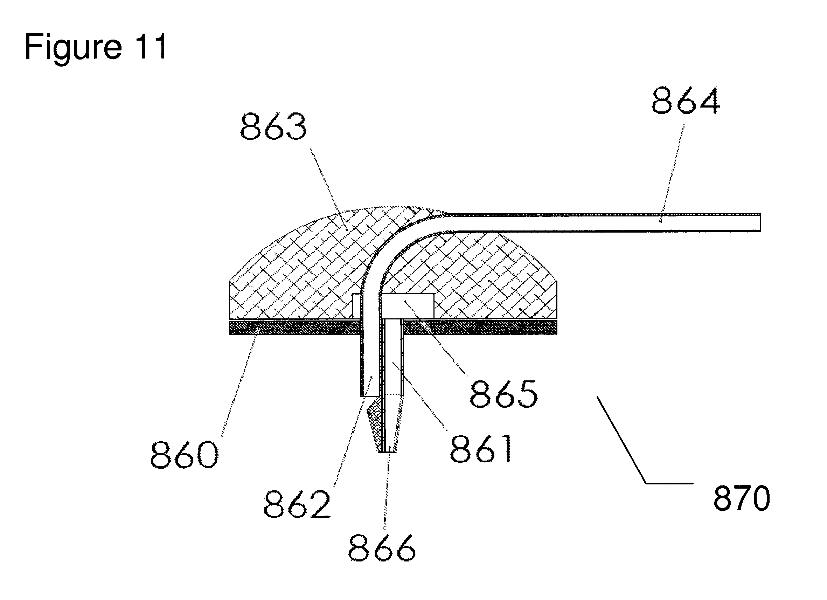

Figure 11 depicts an embodiment of the present invention wherein the substance sensing device includes infusion of additional substance. Substance sensor apparatus 870 includes substance sensor 866, compartment 865, cover 863 and treatment element catheter 862. Additional substances for example including a medicament, chemical, or the like may be infused into the measured tissue region through catheter 862 situated adjacent to substance sensor 866.

Treatment element catheter 862 and substance sensor 866 may be housed in a single double-lumen catheter including a plurality of openings and spanning the same tissue region. Treatment element catheter 862 and substance sensor 866 may be located at two separate tissue regions. An auxiliary unit may be attached providing a power source, additional sensors, treatment element or the like.

In some embodiments, the treatment element catheter 862 may introduce chemicals or substances from an auxiliary drug delivery device attached using another connected catheter 864. In some embodiments, control of the treatment element is accomplished based on treatment and sensor information that may be controlled and analyzed by a controller located in compartment 865.

In some embodiments, the additional substance container can be either disposed in the same housing of the substance sensor or in an auxiliary unit or attached to an auxiliary unit. In some embodiments, a combination of the above treatment methods and/or devices can be placed into a single device to improve its operation and efficacy. The additional substance can modify the local response of the tissue in the vicinity of the substance sensor. Alternatively, other additional substances can affect the local FBR process and modify it in a form so as not to further alter the sensor calibration and consequently reduce the number of needed calibrations needed over the substance sensor operation period. In some embodiments, the additional substance, such as capsaicin, can be applied to skin above the measured tissue region. In some embodiments, the additional substance can be applied using known in the art transdermal delivery methods.

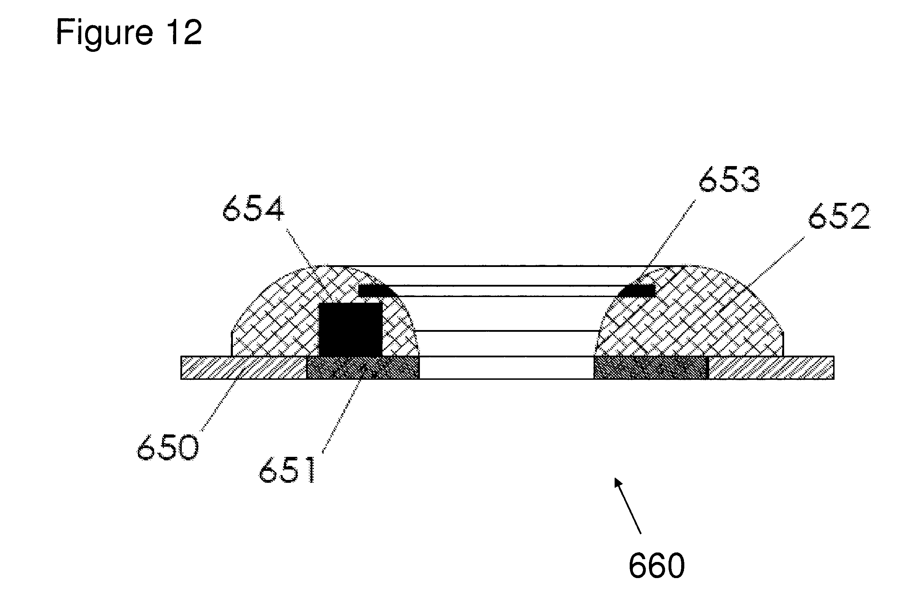

Figure 12 depicts an optional substance sensor apparatus 660 according to some embodiments of the present invention, featuring a cover 652, a compartment 654 (for example for the controller) and a sensor 653. Tissue treatment apparatus 660 is attached to the skin using adhesive 650 and has a circular opening for substance sensor and its securing element. In some embodiments, treatment apparatus 660 can receive a plurality of substance sensors known and accepted in the art. The treatment profile and duration is accomplished according to an algorithm that fits the specific substance sensor 653. The treatment apparatus 660 can also be connected with wire or wirelessly to an auxiliary unit. The treatment apparatus 660 can get the substance readings directly from substance sensor through wired or wireless communication or indirectly through an auxiliary unit and use the readings information in the algorithm to determine the treatment profile.

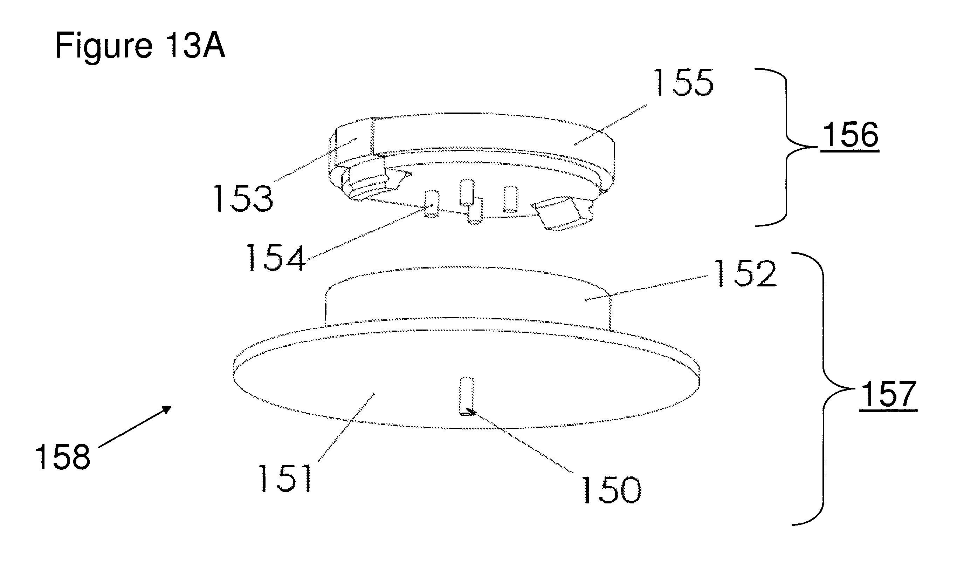

Figures 13A-C show that any of the embodiments of the present invention may be comprised of disposable and reusable portions or any combination thereof. For example, Figure 13A, the substrate sensor apparatus 158 includes a disposable portion 157 and a reusable portion 156. The disposable portion 157 includes substance sensor 150, skin attachment portion 151 and an adaptor mechanism 152 to connect the disposable portion 157 to the reusable portion 156.

In some embodiments, the treatment device (not shown) may be incorporated into either the reusable portion 156 or disposable potion 157. The reusable part 156 may include a processing unit, one or more sensors and a power source. In some embodiments, the power source can be a rechargeable battery (not shown). The disposable portion 157 and reusable portion 156 may be securely coupled with a mechanical locking mechanism 153 and a plurality of pins 154 for electrical connections.

When a rechargeable battery is used, the user may have two alternating reusable portions 156, so that if one is attached to the substance sensor apparatus 158, the other is recharging. When the battery in the substance sensor apparatus 158 is empty, damaged or the user is instructed to remove it (based on a specific battery schedule), the user may switch between the two reusable portions 156. The charger unit has the same mechanical and electrical connection as the disposable part 157 allowing secure and easily fit with the reusable unit 156.

In some embodiments, the reusable part 156 communicates via a communication channel with the auxiliary unit, using wired, wireless, wireline, cellular, optical, IR, RF, or any other communication protocols known and accepted in the art. The treatment device has no communication with other units.

The reusable part or the disposable part is connected with an electrical cable to an auxiliary unit that may include the power source, the control unit or other electronic parts of the device. In some embodiments, a single part disposable treatment device is electrically connected to the auxiliary unit.

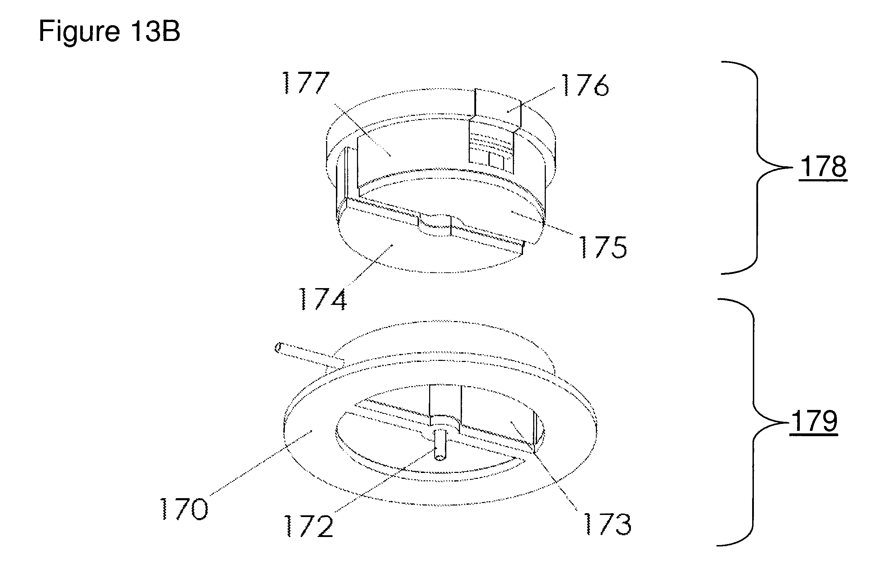

Figure 13B illustrates an alternate embodiment in which the disposable part 179 includes the substance sensor 172, the insertion mechanism and the skin adhering element 170. Prior to insertion of the substance sensor into the tissue, the disposable portion 179 can be attached to the reusable portion 178. Reusable portion 178 includes treatment elements 174 and 175 that contact the skin when the treatment device is attached to the user's skin (not shown).

Disposable part 179 can be attached to the reusable part 178 with a locking mechanism 176. Rim 177 slides into slot 173 upon attachment. The reusable portion 178 can be wired or wirelessly connected to an auxiliary unit. Alternatively, it may not be connected to any additional unit and thus, may include a power source and processing unit. The reusable portion 178 including the treatment element can perform treatments for example heat, suction or the like.

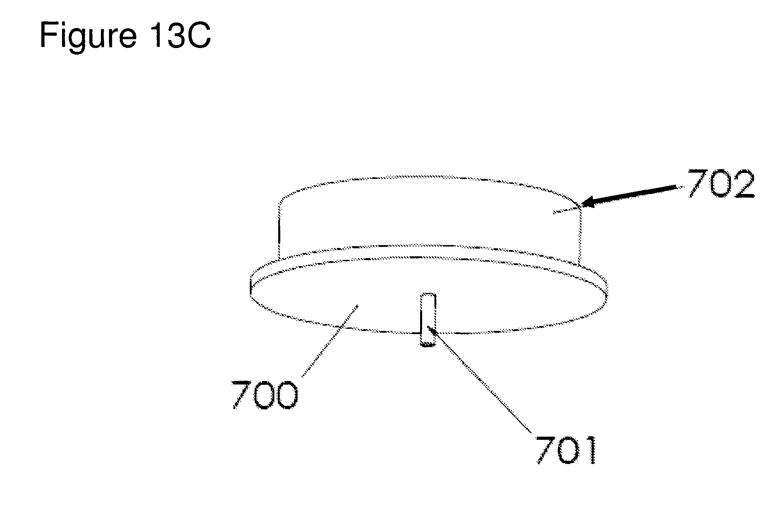

Figure 13C depicts a further optional embodiment of the present invention, wherein the whole unit is disposable, shown as a unit 702 including the substance sensor 701, the treatment device (not shown), the insertion mechanism (not shown), the skin adhering element 700, the power source and processing unit.

Figure 14A-B depicts optional treatment elements, heating elements according to the present invention. Figure 14A depicts a U-shaped heater 34. In this example, the heater 34 is schematically U shaped and attached to the skin around the substance sensor 30. The advantages of this configuration are that the heater is an independent unit that fits many of the commercial substance sensors. The U shaped heater 34 can be thin or thicker and be built in any of many different ways known in the art. The U shaped heater 34 can be made of heat conducting metal with a resistor for heating and a temperature sensor for controlling the temperature. A cable 31 is attached to U shaped heater 34 for providing power.

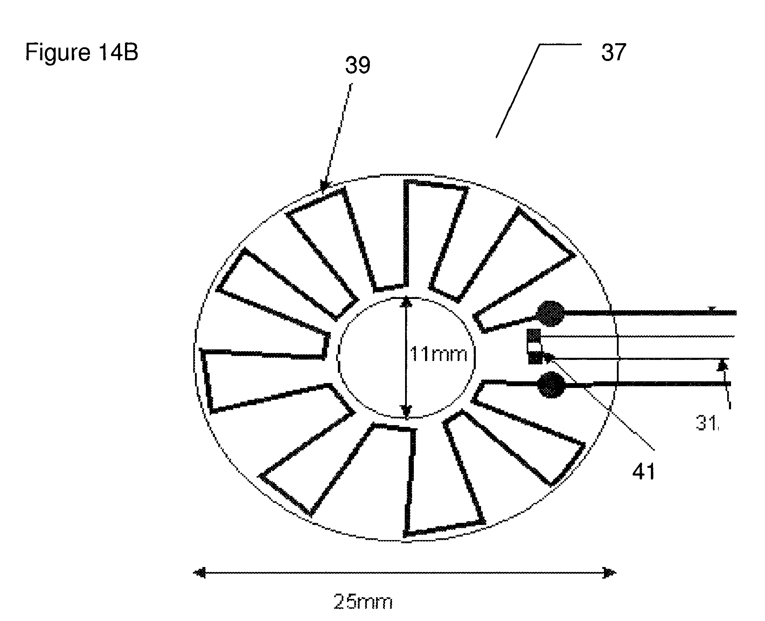

Figure 14B depicts another optional heater structure. In this case, the heater 37 is circular and attached to the substance sensor securing element around the substance sensor prior to insertion into the body, as described above. The shape of the cuts 39 enable attachment of the heater to the substance prior to removing the sensor tip cover, although the sensor tip cover diameter may be larger then the central opening. It is important to remove the substance sensor cover or cup as the last operation before insertion of the substance sensor to the tissue because of safety and sterility issues. However, having the cuts 39 of the heater enables use of a heater 37 with an optimized central opening diameter without the limitations of the cover. In some embodiments, the heating elements (not shown) are placed some distance from the substance sensor tip to optimize the heating of the measured tissue vicinity on one hand and keep the thermal insulation between the substance sensor tip and the heater 37 on the other hand. The heater 37 can be an independent unit that fits many of the commercial substance sensors. The heater 37 includes also a temperature sensor for controlling the temperature, such as thermistors 41. In some embodiments, the thickness of this heater may be about 0.2mm.

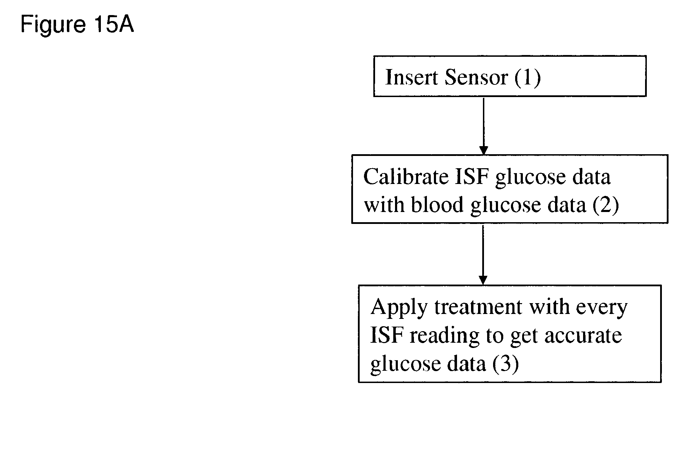

Figure 15A shows an exemplary, illustrative method for combining treatment with a treatment element and ISF measurements, in order to increase the accuracy of such measurements.

As shown, in stage 1, the sensor is inserted to the body tissue; it may be inserted to any location as described herein. In stage 2, the ISF glucose data is calibrated with the blood glucose data. In this stage the tissue treatment is and applied as part of the calibration process to get better accuracy of the sensor's reading and/or shorten the calibration period. In this stage the transport coefficients, for example g, τ, k or the like, and/or one or more equivalent parameters, are calculated. In stage 3, the required treatment and also the treatment level are applied with each ISF reading, and more all ISF readings, to obtain more accurate measurements.

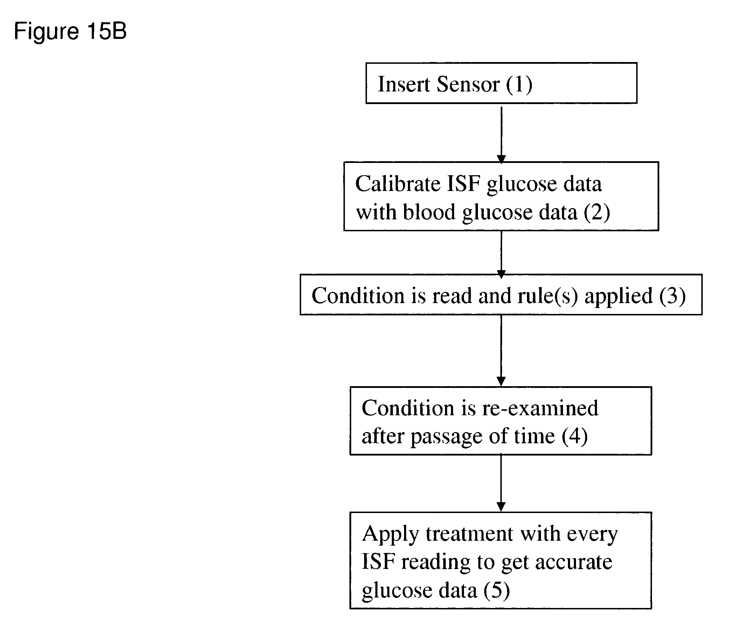

Figure 15B shows another exemplary embodiment of an illustrative method for combining treatment with a treatment element and ISF measurements, as well as with personal calibration, in order to increase the accuracy of such measurements. As shown, stage 1 proceeds as for Figure 15A. In stage 2, the ISF glucose data is calibrated with the blood glucose data before, during or after application of a treatment. In stage 3, at least one of the following conditions are examined: glucose level is below a certain threshold (for instance 80mg/dl), glucose variation (first derivative) is larger than a certain threshold (for instance 2mg/dl/min), glucose change rate starts to change in a rate larger than (second derivative) a certain threshold per time unit (for instance in case of carbohydrate intake or beginning of physical activity), the time of the day at a certain range, the sensor's temperature and/or the temperature of the tissue in the vicinity of the sensor and/or the skin temperature and/or ambient temperature is increased or decreased above or below a certain threshold, reading of additional sensors such as motion detector or physical activity level sensor or blood perfusion sensor get to a specific range or set of ranges. If at least one or a combination of those conditions are met according to a predetermined set of rules the treatment is started. In stage 4, the former conditions are examined again with a different set of rules and thresholds, after the passage of time from the beginning of the treatment. The passage of time itself is also a parameter. If at least one or a combination of those conditions are met according to a predetermined same or different set of rules the treatment is stopped. the process then returns to stage 3.

Stage 5 is performed as for Figure 15A. Stage 5 include stages 3 and 4 inside, such that each time an ISF reading should be taken the conditions are read and re-examined. Stage 5 may be performed before stage 4. Stage 5 is canceled from the process and stage 3 follows stage 4, as discussed above.

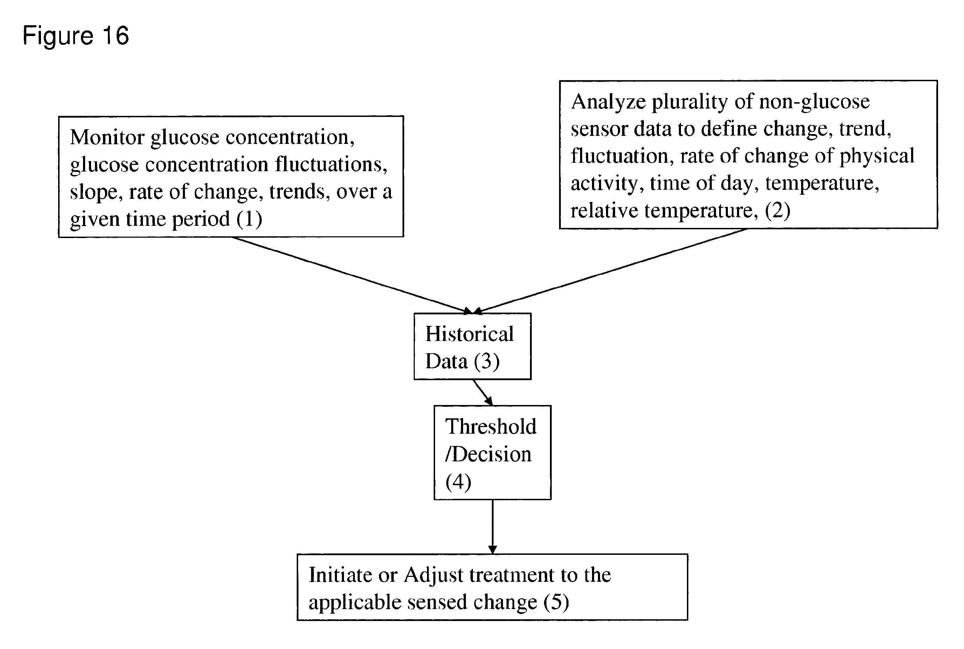

Figure 16 shows an exemplary, illustrative method for combining treatment with a treatment element and ISF measurements, in order to increase the accuracy of such measurements. As shown, in stage 1, the sensors as part of the CGMS system according to some embodiments relate to ISF glucose data to a processor that analyzes the data to obtain trends, changes over time, fluctuations, slope, instantaneous data or the like to while comparing the changing data. In a parallel process, defined in stage 2, non glucose data for example one or more of temperature, relative temperature, time of day, circadian rhythms, physical activity data or the like non glucose information is similarly analyzed for changes, trends, fluctuations, over time. The data and also trends are compared to stored historical data in stage 3, analyzed according to a database of historical information to define how the body reacts to different treatments in the given sensed information. In stage 4, a decision making process is initiate and including a threshold to determine if a treatment protocol and which treatment protocol is to be used. In stage 5, the treatment is changed relative to the sensed and analyzed data.

In some embodiments, some of the treatments described by the present disclosure increases the local blood perfusion around the substance sensor's tip and by that can modify the FBR process and cause it to stabilize earlier leading to fewer changes of the sensor calibration and consequently to a reduced number of needed calibrations over the substance sensor operation period.

In some embodiments, shown in the Figures, the substance sensor is drawn at a 90° penetration angle. As can be understood, other angles are possible. Smaller angles can improve attachment of the substance sensor, but insertion at such angles may be more irritating to the patient.

In some embodiments, an additional sensor for detection if the substance sensor is properly secured to the tissue can be added to the treatment device configuration. Alternatively, it can be added to general subcutaneous substance sensors, such as continuous glucose monitoring sensors mentioned above, and can be used to aid in detecting if the substance sensor securing element is lifted away from or starting to peel off the skin. The sensor can be disposed underneath the substance sensor securing element so that it is in direct contact with the skin, indirect contact through the adhesive layer or other layers attached to the skin. The sensor can measure pressure or skin conductivity, impedance, and/or back-reflected optical or acoustic signal from the skin. A change of the contact level between the sensor and the skin will induce an electronic signal to either the treatment device or to an auxiliary unit. Then, the device can either inform the user to fix the attachment of the securing element to the skin or to reinsert the substance sensor into the tissue in case it is detached. In some embodiments, tissue treatment can be paused or stopped till the substance sensor positioning is fixed. In some embodiments, where the substance sensor readings are used for controlling anther process, such as insulin delivery, this process can be paused or stopped till the substance sensor positioning is fixed.

In some embodiments, the treatment device can be secured to the patient using a strap or a belt that holds the treatment device into its position. The strap can be placed around any part of the patient's body, depending on the location of the measured tissue region and the patient's comfort. Using such a strap can reduce the chances of the substance sensor to be pulled out in more demanding situations, such as jogging. For example, the strap can be placed around the abdomen, leg, thigh, arm etc. In some embodiments, the strap can have a compartment, a pocket or an adaptor for holding the second unit. In embodiments using an auxiliary unit that supports the treatment device, the auxiliary unit can be attached to the strap or even be embedded into the strap. The auxiliary unit can be embedded into the strap or belt, and may be connected to the substance sensor disposable unit by electrical wires using a connector at the wire end. In some embodiments, the auxiliary unit can be attached to the strap and connected to the substance sensor disposable unit. In some embodiments, the disposable unit can be attached to the strap to further reduce chances of the substance sensor being pulled in more demanding situations.

In some embodiments, the power source can be a thin battery, such as the batteries manufactured by Power Paper Ltd. The electronics can be implemented on a flexible printed circuit known in the art to provide the required flexibility for the patient's comfort.

Although particular embodiments have been disclosed herein in detail, this has been done by way of example and for purposes of illustration only, and is not intended to be limiting.

The following documents along with other documents are hereby made of reference Midttun et. al. "Heat washout: a new method for measuring cutaneous blood flow rate in areas with and without arteriovenous anastomoses", Clin. Physiol. 16(3) 259-74 (1996), and Clarke, W.L., et al, Diabetes Care, Volume 28(10), Oct 2005. As can be understood by one skilled in the art, such documents are provided here for illustrative purposes and are not intended to limit the scope of invention.

## Claims

1. An apparatus for measuring an interstitial fluid ("ISF") in a tissue, comprising:
a substance sensor arranged to be in fluid communication with the ISF during use of the apparatus for measuring a level of a substance, wherein the substance sensor is configured to continuously measure a level of the ISF to enable an accurate estimation of the level of the substance;
a treatment element configured to be in contact with tissue in which the substance sensor is arranged and/or tissue that is adjacent thereto and at least one or more additional sensors;
wherein application of treatment by the treatment element to the tissue is configured to improve measurements of the substance levels.
a controller capable of communicating with the substance sensor, the treatment element, and the at least one or more additional sensors, wherein the at least one or more additional sensors as configured to provide data to the controller, **characterised in that** the controller is configured to control activity of the treatments clement by analyzing the substance sensor measurements of the substance and the additional sensor data.

2. The apparatus according to claim 1, wherein the substance comprises glucose.

3. The apparatus according to claim 1, wherein the treatment clement further provides an energy source capable of supplying energy in the form selected from the group consisting of: radiation, mechanical vibrations, pressurization, suction, massaging, acoustic stimulation, magnetic field, electrical stimulation and RF (radiofrequency) energy, light,
and any combination of the foregoing.

4. The apparatus according to claim 1, further comprising a cover and an adhesive layer on the cover for adhering to the skin, wherein the treatment element is provided within at least a portion of the cover and wherein the treatment element comprises a heating element and the heating element comprises a thin disk.

5. The apparatus according to claim 2, further comprising an insulin pump for infusion of insulin in response to measurement of a level of glucose.

6. The apparatus according to claims 5, wherein the pump is configured to infuse the insulin in the vicinity of the sensor.

7. The apparatus according to claim 1, further comprising a catheter inserted to the tissue for receiving the ISF, wherein the sensor is disposed within the catheter.

8. The apparatus according to claim 1, further comprising a fluid pump for pumping the ISF to the sensor.

9. The apparatus according to claim 1, further comprising a display in communication with the controller for displaying at least one of a treatment level and the level of the substance.

10. The apparatus according to claim 9, wherein the treatment comprises application of heat and the treatment level is temperature.

11. The apparatus according to claim 10, wherein the heat is produced by a heating element embedded into the tissue.

12. The apparatus according to claim 1, wherein at least one element is disposable and at least one element is reusable.

13. The apparatus according to any of claims 1-12, wherein the treatment element is configured to perform at least one or more actions selected from a group consisting of: reducing the plasma to ISF delay, reducing the variability of the delay, reducing the time to steady state in tissue where the sensor is placed, stabilizing the calibration process, stabilizing the calibration period, reducing calibration period, minimizing the error of ISF sensors, stabilizing the transport coefficients between the different compartments and stabilizing the parameters relative to the ISF substance levels and any combination thereof.

14. The apparatus according to any of claims 1-13, wherein the treatment clement is configured to apply treatment having an effect selected from a group consisting of: reducing the plasma to ISF delay, reducing the variability of the delay, reducing the time to steady state in tissue where the sensor is placed, stabilizing the calibration process, stabilizing the calibration period, reducing calibration period, minimizing the error of ISF sensors, stabilizing the transport coefficients between the different compartments and stabilizing the parameters relative to the ISF substance levels, increasing vasodilatation, improving diffusion, across blood barrier, improving capillary permeability, improving capillary diffusion, improving cellular metabolism, improving tissue metabolism, improving local solubility, changing local membrane microstructure, improving local permeability, improving facilitative diffusion, improving carrier protein metabolism, improving diffusion mediated by a carrier protein, changing local pressure gradient, changing the local ionic gradient, up-regulating or down-regulating local gene expression, up- regulating or down-regulating local transcription, up-reguhnting or down-regulating local translation, changing local enzymatic activity, changing local lymphatic activity; and improving a Foreign Body Response.

## Patentansprüche

1. Eine Vorrichtung zum Messen einer interstitiellen Flüssigkeit ("ISF") in einem Gewebe, umfassend:
einen Stoffsensor, der angeordnet ist, um mit der ISF während der Verwendung der Vorrichtung zum Messen einer Konzentration eines Stoffes in flüssiger Kommunikation zu sein, wobei der Stoffsensor konfiguriert ist, um kontinuierlich eine Konzentration der ISF zu messen, um eine akkurate Schätzung der Konzentration des Stoffes zu ermöglichen;
ein Behandlungselement, das konfiguriert ist, um mit Gewebe in Kontakt zu sein, in welchem der Stoffsensor angeordnet ist, und/oder Gewebe, das damit benachbart ist, und mindestens einen oder mehrere zusätzliche Sensoren; wobei die Anwendung der Behandlung durch das Behandlungselement auf das Gewebe konfiguriert ist, um die Messung der Stoffkonzentrationen zu verbessern;
eine Steuerung, die mit dem Stoffsensor, dem Behandlungselement und dem mindestens einen oder den mehreren zusätzlichen Sensoren kommunizieren kann, wobei der mindestens eine oder die mehreren zusätzlichen Sensoren konfiguriert sind, um der Steuerung Daten bereitzustellen, **dadurch gekennzeichnet, dass** die Steuerung konfiguriert ist, um Aktivität des Behandlungselements zu steuern, indem sie die Stoffsensormessungen des Stoffes und die zusätzlichen Stoffsensordaten analysiert.

2. Vorrichtung nach Anspruch 1, wobei der Stoff Glucose umfasst.

3. Vorrichtung nach Anspruch 1, wobei das Behandlungselement ferner eine Energiequelle bereitstellt, die Energie in der Form zuführen kann, die ausgewählt ist aus der Gruppe, bestehend aus: Strahlung, mechanische Schwingungen, Druckbeaufschlagung, Saugwirkung, Massieren, akustische Stimulation, magnetisches Feld, elektrische Reizung und RF(Radiofrequenz)-Energie, Licht und eine beliebige Kombination aus dem Vorhergehenden.

4. Vorrichtung nach Anspruch 1, ferner umfassend eine Bedeckung und eine Klebeschicht auf der Bedeckung zum Kleben auf die Haut, wobei das Behandlungselement in mindestens einem Abschnitt der Bedeckung bereitgestellt ist und wobei das Behandlungselement ein Heizelement umfasst und das Heizelement eine dünne Scheibe umfasst.

5. Vorrichtung nach Anspruch 2, ferner umfassend eine Insulinpumpe zur Infusion von Insulin als Reaktion auf die Messung einer Konzentration an Glucose.

6. Vorrichtung nach Anspruch 5, wobei die Pumpe konfiguriert ist, um das Insulin in der Nähe des Sensors zu infundieren.

7. Vorrichtung nach Anspruch 1, ferner umfassend einen Katheter, der in das Gewebe eingeführt wird, um die ISF aufzunehmen, wobei der Sensor innerhalb des Katheters befindlich ist.

8. Vorrichtung nach Anspruch 1, ferner umfassend eine Flüssigkeitspumpe zum Pumpen der ISF zu dem Sensor.

9. Vorrichtung nach Anspruch 1, ferner umfassend eine Anzeige, die mit der Steuerung in Kommunikation ist, um mindestens entweder den Behandlungsgrad und/oder die Stoffkonzentration anzuzeigen.

10. Vorrichtung nach Anspruch 9, wobei die Behandlung die Anwendung von Hitze umfasst und der Behandlungsgrad Temperatur ist.

11. Vorrichtung nach Anspruch 10, wobei die Hitze durch ein in dem Gewebe eingebettetes Heizelement erzeugt wird.

12. Vorrichtung nach Anspruch 1, wobei mindestens ein Element wegwerfbar ist und mindestens ein Element wiederverwendbar ist.

13. Vorrichtung nach einem der Ansprüche 1-12, wobei das Behandlungselement konfiguriert ist, um mindestens eine oder mehrere Tätigkeiten durchzuführen, die ausgewählt sind aus einer Gruppe, bestehen aus: Reduzieren der Plasma-ISF-Verzögerung, Reduzieren der Variabilität der Verzögerung, Reduzieren der Zeit bis zum aufrechterhaltenen Ungleichgewicht im Gewebe, wo der Sensor platziert ist, Stabilisieren des Kalibrierungsvorgangs, Stabilisieren der Kalibrierungsperiode, Reduzieren der Kalibrierungsperiode, Minimieren des Fehlers von ISF-Sensoren, Stabilisieren der Transportkoeffizienten zwischen den verschiedenen Kammern und Stabilisieren der Parameter relativ zu den ISF-Stoffkonzentrationen und eine beliebige Kombination davon.

14. Vorrichtung nach einem der Ansprüche 1-13, wobei das Behandlungselement konfiguriert ist, um eine Behandlung mit einer Wirkung anzuwenden, die ausgewählt ist aus der Gruppe, bestehend aus: Reduzieren der Plasma-ISF-Verzögerung, Reduzieren der Variabilität der Verzögerung, Reduzieren der Zeit bis zum aufrechterhaltenen Ungleichgewicht im Gewebe, wo der Sensor platziert ist, Stabilisieren des Kalibrierungsvorgangs, Stabilisieren der Kalibrierungsperiode, Reduzieren der Kalibrierungsperiode, Minimieren des Fehlers von ISF-Sensoren, Stabilisieren der Transportkoeffizienten zwischen den verschiedenen Kammern und Stabilisieren der Parameter relativ zu den ISF-Stoffkonzentrationen, Erhöhen der Blutgefäßerweiterung, Verbessern der Diffusion über die Blut-Schranke, Verbessern der Kapillarpermeabilität, Verbessern der Kapillardiffusion, Verbessern des Zellstoffwechsels, Verbessern des Gewebestoffwechsels, Verbessern der lokalen Löslichkeit, Ändern der lokalen Membranmikrostruktur, Verbessern der lokalen Permeabilität, Verbessern der erleichterten Diffusion, Verbessern des Transportproteinstoffwechsels, Verbessern der durch ein Transportprotein vermittelten Diffusion, Ändern des lokalen Druckgradienten, Ändern des lokalen Ionengradienten, Hochregeln oder Herunterregeln der lokalen Genexpression, Hochregeln oder Herunterregeln der lokalen Transkription, Hochregeln oder Herunterregeln der lokalen Translation, Ändern der lokalen enzymatischen Aktivität, Ändern der lokalen lymphatischen Aktivität; und Verbessern einer Fremdkörperreaktion.

## Revendications

1. Un appareil pour mesurer un liquide interstitiel (« ISF ») dans un tissu, comprenant :
un détecteur de substance agencé pour être en communication fluidique avec l'ISF durant l'utilisation de l'appareil pour mesurer un niveau d'une substance, dans lequel le détecteur de substance est configuré pour mesurer de façon continue un niveau de l'ISF pour permettre une estimation précise du niveau de la substance ;
un élément de traitement configuré pour être en contact avec du tissu dans lequel le détecteur de substance est agencé et/ou du tissu qui est adjacent à ce dernier et au moins un ou plusieurs détecteurs supplémentaires ; dans lequel l'application de traitement par l'élément de traitement au tissu est configurée pour améliorer la mesure des niveaux de substance ;
un contrôleur capable de communiquer avec le détecteur de substance, l'élément de traitement, et les au moins un ou plusieurs détecteurs supplémentaires, dans lequel les au moins un ou plusieurs détecteurs supplémentaires sont configurés pour fournir des données au contrôleur, **caractérisé en ce que** le contrôleur est configuré pour contrôler l'activité de l'élément de traitement en analysant les mesures de la substance par le détecteur de substance et les données des détecteurs supplémentaires

2. L'appareil selon la revendication 1, dans lequel la substance comprend du glucose.

3. L'appareil selon la revendication 1, dans lequel l'élément de traitement fournit en outre une source d'énergie capable de fournir de l'énergie sous la forme sélectionnée dans le groupe composé de : rayonnement, vibrations mécaniques, pressurisation, succion, massage, stimulation acoustique, champ magnétique, stimulation électrique et énergie RF (radiofréquence), lumière, et n'importe quelle combinaison de ce qui précède.

4. L'appareil selon la revendication 1, comprenant en outre un couvercle et une couche adhésive sur le couvercle pour adhérer à la peau, dans lequel l'élément de traitement est fourni à l'intérieur d'au moins une portion du couvercle et dans lequel l'élément de traitement comprend un élément de chauffage et l'élément de chauffage comprend un disque fin.

5. L'appareil selon la revendication 2, comprenant en outre une pompe à insuline pour la perfusion d'insuline en réponse à la mesure d'un niveau de glucose.

6. L'appareil selon la revendication 5, dans lequel la pompe est configurée pour perfuser l'insuline dans le voisinage du détecteur.

7. L'appareil selon la revendication 1, comprenant en outre un cathéter inséré dans le tissu pour recevoir l'ISF, dans lequel le détecteur est disposé à l'intérieur du cathéter.

8. L'appareil selon la revendication 1, comprenant en doute une pompe à liquide pour pomper l'ISF jusqu'au détecteur.

9. L'appareil selon la revendication 1, comprenant en outre un affichage en communication avec le contrôleur pour afficher au moins un niveau parmi un niveau de traitement et le niveau de la substance.

10. L'appareil selon la revendication 9, dans lequel le traitement comprend l'application de chaleur et le niveau de traitement est la température.

11. L'appareil selon la revendication 10, dans lequel la chaleur est produite par un élément de chauffage intégré dans le tissu.

12. L'appareil selon la revendication 1, dans lequel au moins un élément est jetable et au moins un élément est réutilisable.

13. L'appareil selon n'importe lesquelles des revendications 1 à 12, dans lequel l'élément de traitement est configuré pour réaliser au moins une ou plusieurs actions sélectionnées dans un groupe composé de : réduire le délai du plasma à l'ISF, réduire la variabilité du délai, réduire le temps jusqu'à l'état stable dans le tissu où le détecteur est placé, stabiliser le processus d'étalonnage, stabiliser la période d'étalonnage, réduire la période d'étalonnage, minimiser l'erreur des détecteurs d'ISF, stabiliser les coefficients de transport entre les différents compartiments et stabiliser les paramètres relatifs aux niveaux de substance de l'ISF et n'importe quelle combinaison de ces derniers.

14. L'appareil selon n'importe lesquelles des revendications 1 à 13, dans lequel l'élément de traitement est configuré pour appliquer un traitement ayant un effet sélectionné dans un groupe composé de : réduire le délai du plasma à l'ISF, réduire la variabilité du délai, réduire le temps jusqu'à l'état stable dans le tissu où le détecteur est placé, stabiliser le processus d'étalonnage, stabiliser la période d'étalonnage, réduire la période d'étalonnage, minimiser l'erreur des détecteurs d'ISF, stabiliser les coefficients de transport entre les différents compartiments et stabiliser les paramètres relatifs aux niveaux de substance de l'ISF, augmenter la vasodilatation, améliorer la diffusion à travers la barrière sanguine, améliorer la perméabilité capillaire, améliorer la diffusion capillaire, améliorer le métabolisme cellulaire, améliorer le métabolisme des tissus, améliorer la solubilité locale, modifier la microstructure membranaire locale, améliorer la perméabilité locale, améliorer la diffusion facilitatrice, améliorer le métabolisme des protéines porteuses, améliorer la diffusion par la médiation d'une protéine porteuse, modifier le gradient de tension locale, modifier le gradient ionique local, réguler à la hausse ou réguler à la baisse l'expression génétique locale, réguler à la hausse ou réguler à la baisse la transcription locale, réguler à la hausse ou réguler à la baisse la translation locale, modifier l'activité enzymatique locale, modifier l'activité lymphatique locale, et améliorer une réaction aux corps étrangers.
